# EUROPEAN PATENT APPLICATION

(11) **EP 3 603 612 A1**
(43) Date of publication of application: **05.02.2020**
(21) Application number: 18771749.1
(22) Date of filing: 23.03.2018
(51) Int. Cl.: A61K 8/44, A61K 31/198, A61K 31/405, A61K 31/4172, A61P 17/00, A61P 17/16, A61P 43/00, A61Q 1/02, A61Q 17/04, A61Q 19/00, A61Q 19/08, A61Q 19/10, G01N 27/62, G01N 30/72, G01N 30/88, G01N 33/68

(54) **STRATUM CORNEUM FUNCTION IMPROVING AGENT**

(30) Priority: 24.03.2017 JP 2017060069
(71) Applicant: Ajinomoto Co., Inc., Tokyo 104-8315 (JP)
(72) Inventor: TAKINO, Yoshinobu, Kawasaki-shi Kanagawa 210-8681 (JP); OKURA, Fumie, Kawasaki-shi Kanagawa 210-8681 (JP); KARAKAWA, Sachise, Kawasaki-shi Kanagawa 210-8681 (JP); IKEGAMI, Eri, Kawasaki-shi Kanagawa 210-8681 (JP)
(74) Representative: Mewburn Ellis LLP
(86) International application number: PCT/JP2018/011910
(87) International publication number: WO 2018/174286

(57) **Abstract**

Provided is a horny layer function improving agent containing at least one D-amino acid selected from the group consisting of D-arginine, D-histidine, D-leucine, D-tyrosine, D-valine, D-allo-threonine, D-lysine, D-tryptophan, D-allo-isoleucine, D-asparagine, D-phenylalanine, and D-glutamine as an active ingredient.

## Description

### Field

The present invention relates to a horny layer function improving agent that can exhibit functions such as moisture retainability that retains water thereinside, barrier function that inhibits the entry of foreign substances such as viruses and bacteria from the outside, and tiny wrinkle preventing or improving ability in a horny layer.

### Background

The horny layer has functions such as moisture retainability that retains water thereinside and barrier function that inhibits the entry of foreign substances such as viruses and bacteria from the outside. Biological components such as sebum, an intracellular lipid, and a natural moisturizing factor are known as substances that act on these functions.

Profilaggrin stored within a granular layer of skin tissue is decomposed into filaggrin through dephosphorylation during keratinization. Filaggrin moves from the granular layer to the horny layer, binds to keratin fiber and aggregates, and forms a keratin pattern. Filaggrin is then decomposed into a low molecular weight compound such as urocanic acid to be a natural moisturizing factor related to water retention and ultraviolet ray absorption. For this reason, it is considered that more promoted production of filaggrin increases the amount of the natural moisturizing factor as the decomposed product thereof and improves the moisture retainability of the skin.

It is said that the formation of wrinkles as one of the representative aging changes of the skin begins with tiny wrinkle formation caused by dryness of the skin. For this reason, it is considered that when the production of filaggrin is promoted to improve the moisture retainability of the skin, dryness is inhibited, and the formation of tiny wrinkles can be inhibited.

Factors forming the horny layer such as corneocytes and an intercellular lipid present among the corneocytes inhibit the entry of foreign substances such as viruses and bacteria from the outside. Given these circumstances, when conditions including the number and size of the corneocytes are insufficient, gaps occur in the horny layer, and barrier function declines.

Around the corneocytes embracing a structure in which keratin such as keratin 10 and filaggrin aggregation, there is a marginal zone, which is an extremely strong, huge insoluble structure that backs cell membranes of the corneocytes. The main structural elements of the marginal zone are proteins such as involucrin produced in prickle cells and loricrin produced in granular cells, which are cross-linked with enzymes such as transglutaminase (TG) 1. Given these circumstances, it is considered that filaggrin, involucrin, transglutaminase 1, keratin 10, and loricrin have a strong influence on the formation of the corneocytes and relate to the barrier function of the skin.

It is reported that the expression of filaggrin, involucrin, transglutaminase 1, keratin 10, and loricrin is promoted by specific peptides such as glycylproline (Patent Literature 1) and a Piper longum extract (Patent Literature 2) .

D-amino acid compounds such as D-alanine and D-hydroxyproline (Patent Literature 3) are reported as compounds that promote the production of laminin 332 as a protein that is present in a large amount in a basement membrane that separates epidermis and dermis and is considered to play an important role in the structure and function of the skin.

A technique containing a D-amino acid such as D-lysine hydrochloride and a chelating agent is reported as a skin external preparation that has effects such as a moisture retaining effect and does not produce degeneration such as a change of smell and a change of color (refer to Patent Literature 4). A technique containing at least one compound selected from D-glutamic acid and a derivative and/or a salt thereof is reported as a stable, safe oral composition that acts on skin barrier function and can reduce and/or prevent skin roughness(refer to Patent Literature 5).

### Citation List

### Patent Literature

Patent Literature 1: WO 2014/175001
Patent Literature 2: Japanese Patent Application Laid-open No. 2012-232920
Patent Literature 3: WO 2011/040082
Patent Literature 4: Japanese Patent Application Laid-open No. 2012-006870
Patent Literature 5: WO 2011/040185

### Summary

### Technical Problem

However, both the specific peptide such as glycylproline and the Piper longum extract are weak in expression promotion action of moisture retention-related proteins such as filaggrin and are unsatisfactory for use as an active ingredient of moisture retaining agents.

Patent Literature 3 discloses that the laminin 332 production promotion effect is not expressed when a D-amino acid compound such as D-aspartic acid is used. In addition, it does not describe nor suggest the activity of proteins other than laminin 332 in the skin.

In Patent Literature 4, D-lysine is used in order to inhibit degeneration such as a change of smell and a change of color when the chelating agent is used, and the moisture retaining effect is expressed in both compounds of D-lysine and L-lysine. Consequently, it is not concluded that only D-lysine in the racemic body selectively has an influence on the moisture retainability. In addition, the moisture retaining effect of Patent Literature 4 is determined only by the subjectivity of subjects.

Patent Literature 4 states that D-glutamic acid reduces a TEWL value when orally administered. However, it does not describe nor suggest the expression of the effect through other administration forms (e.g., skin care products).

In addition, Patent Literature 4 and Patent Literature 5 do not describe nor suggest the potential to improve both abilities of moisture retainability and barrier function.

The present invention has been made in view of the above problems, and an object thereof is to provide a horny layer function improving agent that can exhibit functions such as moisture retainability that retains water thereinside, barrier function that inhibits the entry of foreign substances such as viruses and bacteria from the outside, and tiny wrinkle preventing or improving ability in a horny layer.

### Solution to Problem

The present inventors diligently have studied to achieve the above object. As a result, the present inventors have found that specific D-amino acids promote the production of a moisture retainability-related protein (filaggrin), a barrier function-related protein (filaggrin, involucrin, transglutaminase 1, keratin 10, and loricrin), a protein for tiny wrinkle preventing or improving (filaggrin), and reached the present invention based on such findings.

That is, the present invention is as follows.
[1] A horny layer function improving agent comprising at least one D-amino acid selected from the group consisting of D-arginine, D-histidine, D-leucine, D-proline, D-serine, D-tyrosine, D-valine, D-allo-threonine, D-aspartic acid, D-lysine, D-tryptophan, D-allo-isoleucine, D-asparagine, D-phenylalanine, D-alanine, D-glutamine, D-glutamic acid, D-cysteine, D-methionine and D-threonine (hereinafter, in the present specification, they are abbreviated as "D-Arg", "D-His", "D-Leu", "D-Pro", "D-Ser", "D-Tyr", "D-Val", "D-allo-Thr", "D-Asp", "D-Lys", "D-Trp", "D-allo-Ile", "D-Asn", "D-Phe", "D-Ala", "D-Gln", "D-Glu", "D-Cys", "D-Met", "D-Thr", respectively.) as an active ingredient.
[2] The horny layer function improving agent according to the above [1], wherein the horny layer function improving is retention of a horny layer moisture content.
[3] The horny layer function improving agent according to the above [1], wherein
   the D-amino acid is at least one selected from the group consisting of D-Arg, D-His, D-Leu, D-Pro, D-Ser, D-Val, D-allo-Thr, D-Asp, D-Lys, D-Trp, D-allo-Ile, D-Asn, D-Phe, D-Ala, D-Gln, D-Glu, D-Cys, D-Met, and D-Thr, and
   the horny layer function improving is inhibition of transepidermal water loss.
[4] The horny layer function improving agent according to the above [1], wherein
   the D-amino acid is at least one selected from the group consisting of D-His, D-Leu, D-Pro, D-Tyr, D-Asp, D-Lys, D-Trp, D-allo-Ile, D-Asn, D-Phe, D-Ala, D-Gln, D-Glu, D-Cys, D-Met, and D-Thr, and
   the horny layer function improving is a reduction in a wrinkle area rate.
[5] A moisture retainability improving agent comprising at least one D-amino acid selected from the group consisting of D-Arg, D-His, D-Leu, D-Pro, D-Ser, D-Tyr, D-Val, D-allo-Thr, D-Asp, D-Lys, D-Trp, D-allo-Ile, D-Asn, D-Phe, D-Ala, D-Gln, D-Glu, D-Cys, D-Met, and D-Thr as an active ingredient.
[6] The moisture retainability improving agent according to the above [5], wherein the D-amino acid is at least one selected from the group consisting of D-His, D-Leu, D-Lys, D-Trp, D-allo-Ile, D-Asn, and D-Phe.
[7] The moisture retainability improving agent according to the above [5] or [6], wherein the D-amino acid is at least one selected from the group consisting of D-His, D-Leu, D-Lys, D-Trp, D-allo-Ile, and D-Phe.
[8] A barrier function improving agent comprising at least one D-amino acid selected from the group consisting of D-Arg, D-His, D-Leu, D-Pro, D-Ser, D-Val, D-allo-Thr, D-Asp, D-Lys, D-Trp, D-allo-Ile, D-Asn, D-Phe, D-Ala, D-Glu, D-Cys, D-Met, D-Gln, and D-Thr as an active ingredient.
[9] The barrier function improving agent according to the above [8], wherein the D-amino acid is at least one D-amino acid selected from the group consisting of D-His, D-Leu, D-Lys, D-Trp, D-allo-Ile, D-Asn, and D-Phe.
[10] The barrier function improving agent according to the above [8] or [9], wherein the D-amino acid is at least one D-amino acid selected from the group consisting of D-His, D-Leu, D-Lys, D-Trp, D-allo-Ile, and D-Phe.
[11] A tiny wrinkle preventing or improving agent comprising at least one selected from the group consisting of D-His, D-Leu, D-Tyr, D-Asp, D-Lys, D-Trp, D-allo-Ile, D-Asn, D-Phe, D-Ala, D-Glu, D-Gln, D-Cys, D-Met, and D-Thr as an active ingredient.
[12] The tiny wrinkle preventing or improving agent according to the above [11], wherein the D-amino acid is at least one selected from the group consisting of D-Leu, D-Lys, D-Trp, and D-Asn.
[13] The tiny wrinkle preventing or improving agent according to the above [11] or [12], wherein the D-amino acid is at least one selected from the group consisting of D-Leu, D-Lys, and D-Trp.
[14] An expression promoter of a gene of at least one protein selected from filaggrin, involucrin, loricrin, keratin 10, and transglutaminase 1 comprising at least one selected from the group consisting of D-Asp, D-Lys, D-Trp, D-allo-Ile, D-Asn, D-Phe, D-Ala, and D-Glu as an active ingredient.
[15] A filaggrin production promoter comprising at least one selected from the group consisting of D-His, D-Leu, D-Pro, D-Asp, D-Lys, D-Trp, D-allo-Ile, D-Phe, D-Ala, D-Glu, D-Cys, D-Met, and D-Thr as an active ingredient.
[16] A skin cosmetic or skin external preparation comprising the horny layer function improving agent according to any one of the above [1] to [4], the moisture retainability improving agent according to any one of the above [5] to [7], the barrier function improving agent according to any one of the above [8] to [10], the tiny wrinkle preventing or improving agent according to any one of the above [11] to [13], the expression promoter according to the above [14], or the production promoter according to the above [15].
[17] The skin cosmetic or skin external preparation according to the above [16], further comprising at least one base selected from the group consisting of a polyol compound, a surfactant, a higher alcohol, a thickener, a chelating agent, an preservative, and a silicone.
[18] The skin cosmetics or skin external preparation according to the above [16] or [17], wherein a dosage form is milky lotion, cream, skin lotion, or gel.
[19] A method of evaluation of a skin by a D-amino acid, the method comprising:
   measuring at least one selected from the group consisting of a horny layer moisture content, a wrinkle area rate, and a transepidermal water loss of a skin of a subject to determine at least one selected from the group consisting of moisture retainability, tiny wrinkle preventing or improving ability, and barrier function of the skin of the subject;
   collecting a horny layer from a measurement site of the skin of the subject;
   identifying at least one D-amino acid level contained in the horny layer; and
   comparing a relation between the at least one selected from the group consisting of moisture retainability, barrier function, and tiny wrinkle preventing or improving ability of the skin and the D-amino acid level among results of the subject.
[20] The method of evaluation according to the above [19], wherein the identification of the D-amino acid level is quantification of a D-amino acid content per content of protein contained in the horny layer.
[21] The method of evaluation according to the above [19] or [20], wherein the quantification of the D-amino acid content is performed with a liquid chromatograph-tandem mass spectrometer.
[22] The method of evaluation according to the above [20] or [21], wherein measurement of the content of protein is performed by the BCA method.
[23] The method of evaluation according to any one of the above [19] to [22], wherein the D-amino acid comprises at least one selected from the group consisting of D-Arg, D-His, D-Leu, D-Pro, D-Ser, D-Tyr, D-Val, D-allo-Thr, D-Asp, D-Lys, D-Trp, D-allo-Ile, D-Asn, D-Phe, D-Ala, D-Gln, D-Glu, and D-Met.
[24] The method of evaluation according to the above [23], wherein
   the subject is a woman in her twenties to thirties, and
   the D-amino acid is at least one selected from the group consisting of D-Arg, D-His, D-Leu, D-Pro, D-Ser, D-Tyr, D-Val, D-allo-Thr, D-Asp, D-Lys, D-Trp, D-allo-Ile, D-Asn, and D-Phe,
   the method evaluating at least moisture retainability.
[25] The method of evaluation according to the above [23], wherein
   the subject is a woman in her twenties to thirties, and
   the D-amino acid is at least one selected from the group consisting of D-His, D-Leu, D-Pro, D-Ser, D-Val, D-allo-Thr, D-Asp, D-Trp, D-allo-Ile, D-Asn, and D-Phe,
   the method evaluating at least barrier function.
[26] The method of evaluation according to the above [23], wherein
   the subject is a woman in her forties to sixties, and
   the D-amino acid is at least one selected from the group consisting of D-Arg, D-His, D-Leu, D-Pro, D-Val, D-allo-Thr, D-Asp, D-Lys, D-Trp, D-allo-Ile, D-Asn, and D-Gln,
   the method evaluating at least moisture retainability.
[27] The method of evaluation according to the above [23], wherein
   the subject is a woman in her forties to sixties, and
   the D-amino acid is at least one selected from the group consisting of D-Arg, D-His, D-Leu, D-Pro, D-Ser, D-Val, D-allo-Thr, D-Lys, D-Trp, D-allo-Ile, D-Phe, and D-Gln,
   the method evaluating at least barrier function.
[28] The method of evaluation according to the above [23], wherein
   the subject is a woman in her twenties to thirties, and
   the D-amino acid is at least one selected from the group consisting of D-Leu, D-Tyr, D-Asp, D-Lys, D-Trp, D-Asn, and D-Gln,
   the method evaluating at least tiny wrinkle preventing or improving ability.
[29] The method of evaluation according to the above [23], wherein
   the subject is a woman in her forties to sixties, and
   the D-amino acid is at least either D-Asp or D-Lys,
   the method evaluating at least tiny wrinkle preventing or improving ability.
[30] The method of evaluation according to any one of the above [19] to [29], further comprising, as a result of the comparison, when a correlation is determined between at least one selected from the group consisting of the horny layer moisture content, the wrinkle area rate, and the transepidermal water loss of a skin, and the D-amino acid level, selecting the correlation as an indicator of an evaluation of the skin of the subject
[31] A method of evaluation of skin cosmetics or skin external preparation, the method comprising:
   measuring D-amino acid level constituting the correlation selected by the method of evaluation according to the above [19], and contained in a horny layer of a measurement site of a skin of a subject before and after dosing of the skin cosmetics or skin external preparation, or the active ingredient candidate substance thereof; and
   identifying at least one selected from the group consisting of the horny layer moisture content, the wrinkle area rate, and the transepidermal water loss of a skin before and after dosing based on the correlation from the D-amino acid level, and comparing therewith.
[32] A method of evaluation of effectiveness of skin cosmetics or skin external preparation to a subject, the method comprising:
   measuring D-amino acid level constituting the correlation selected by the method of evaluation according to the above [19], and contained in a horny layer of a measurement site of a skin of the subject before and after dosing of the skin cosmetics or skin external preparation; and
   identifying at least one selected from the group consisting of the horny layer moisture content, the wrinkle area rate, and the transepidermal water loss of a skin before and after dosing based on the correlation from the D-amino acid level, and comparing therewith.
   In the present specification, the amino acids may be represented by a conventional three-letter notation (for example, "Asn", "Gln") with a configuration prefix such as "L-", "D-", "D,L-" or the like.

### Advantageous Effects of Invention

The horny layer function improving agent of the present invention can exhibit functions such as moisture retainability that retains water thereinside, barrier function that inhibits the entry of foreign substances such as viruses and bacteria from the outside, and tiny wrinkle preventing or improving ability in a horny layer.

### Brief Description of Drawings

FIG. 1 is a graph of an increase (a relative value to an increase of a control) of a moisture retainability/barrier function/tiny wrinkle preventing or improving ability-related protein gene (filaggrin) of D-Asn (Example 1).
FIG. 2 is a graph of an increase (a relative value to an increase of a control) of a barrier function-related protein gene (involucrin) of D-Asn (Example 1).
FIG. 3 is a graph of an increase (a relative value to an increase of a control) of a barrier function-related protein gene (keratin 10) of D-Asn (Example 1).
FIG. 4 is a graph of an increase (a relative value to an increase of a control) of a barrier function-related protein gene (loricrin) of D-Asn (Example 1).
FIG. 5 is a graph of an increase (a relative value to an increase of a control) of the moisture retainability/barrier function/tiny wrinkle preventing or improving ability-related protein gene (filaggrin) of D-Asp (Example 2).
FIG. 6 is a graph of an increase (a relative value to an increase of a control) of the barrier function-related protein gene (involucrin) of D-Asp (Example 2).
FIG. 7 is a graph of an increase (a relative value to an increase of a control) of the barrier function-related protein gene (keratin 10) of D-Asp (Example 2).
FIG. 8 is a graph of an increase (a relative value to an increase of a control) of the barrier function-related protein gene (loricrin) of D-Asp (Example 2).
FIG. 9 is a graph of an increase (a relative value to an increase of a control) of a barrier function-related protein gene (transglutaminase 1) of D-Asp (Example 2).
FIG. 10 is a diagram of a correlation between a D-Asp level
   in a horny layer and a horny layer moisture content in women in their twenties to thirties.
FIG. 11 is a diagram of a correlation between a D-Phe level in the horny layer and the horny layer moisture content in the women in their twenties to thirties.
FIG. 12 is a diagram of a correlation between a D-allo-Ile level in the horny layer and the horny layer moisture content in women in their forties to sixties.
FIG. 13 is a diagram of a correlation between a D-Asn level in the horny layer and a transepidermal water loss (TEWL) in the women in their twenties to thirties.
FIG. 14 is a diagram of a correlation between a D-Phe level in the horny layer and the transepidermal water loss (TEWL) in the women in their twenties to thirties.
FIG. 15 is a diagram of a correlation between a D-allo-Ile level in the horny layer and the transepidermal water loss (TEWL) in the women in their forties to sixties.
FIG. 16 is a diagram of a correlation between a D-Asn level in the horny layer and a wrinkle area rate in the women in their twenties to thirties.
FIG. 17 is a diagram of a correlation between a D-Gln level in the horny layer and the wrinkle area rate in the women in their twenties to thirties.
FIG. 18 is a diagram of a correlation between a D-Lys level in the horny layer and the wrinkle area rate in the women in their forties to sixties.
FIG. 19 is a graph of cell survival rates in normal human epidermal keratinocytes when D-Asn, D-Asp, D-Lys, D-Phe, D-Trp, D-allo-Ile, D-Ala, and D-Glu are added.
FIG. 20 is a graph of a filaggrin production amount in normal human neonatal epidermal keratinocytes when D-His is added.
FIG. 21 is a graph of the filaggrin production amount in normal human neonatal epidermal keratinocytes when D-Leu is added.
FIG. 22 is a graph of the filaggrin production amount in normal human neonatal epidermal keratinocytes when D-Trp is added.
FIG. 23 is a graph of the filaggrin production amount in normal human neonatal epidermal keratinocytes when D-Ala is added.
FIG. 24 is a graph of the filaggrin production amount in normal human neonatal epidermal keratinocytes when D-allo-Ile is added.
FIG. 25 is a graph of the filaggrin production amount in normal human neonatal epidermal keratinocytes when D-Asp is added.
FIG. 26 is a graph of the filaggrin production amount in normal human neonatal epidermal keratinocytes when D-Glu is added.
FIG. 27 is a graph of the filaggrin production amount in normal human neonatal epidermal keratinocytes when D-Lys is added.
FIG. 28 is a graph of the filaggrin production amount in normal human neonatal epidermal keratinocytes when D-Phe is added.
FIG. 29 is a graph of the filaggrin production amount in normal human neonatal epidermal keratinocytes when D-Pro is added.
FIG. 30 is a graph of the filaggrin production amount in normal human neonatal epidermal keratinocytes when D-Cys is added.
FIG. 31 is a graph of the filaggrin production amount in normal human neonatal epidermal keratinocytes when D-Met is added.
FIG. 32 is a graph of the filaggrin production amount in normal human neonatal epidermal keratinocytes when D-Thr is added.

### Description of Embodiments

In the present specification, when expressed simply as "agent," it collectively refers to a horny layer function improving agent, a moisture retainability improving agent, a barrier function improving agent, a tiny wrinkle preventing or improving agent, a gene expression promoter, and a production promoter.

### [1. Horny Layer Function Improving Agent]

In the present specification, the horny layer function improving agent refers to one that can exhibit any effect of improvement in moisture retainability that increases or retains a horny layer moisture content, improvement in barrier function that inhibits the entry of foreign substances such as viruses and bacteria from the outside, and improvement in tiny wrinkle preventing or improving ability when applied to the skin.

In the present specification, "moisture retainability" refers to retention of the horny layer moisture content; a larger horny layer moisture content indicates more excellent moisture retainability. "Barrier function" refers to inhibition of transepidermal water loss; a smaller transepidermal water loss (hereinafter, also referred to as "TEWL") indicates more excellent barrier function. "Tiny wrinkle preventing or improving ability" is evaluated by a wrinkle area rate; a smaller wrinkle area rate indicates more excellent tiny wrinkle preventing or improving ability.

A horny layer function improving agent of the present invention contains at least one D-amino acid selected from the group consisting of D-Arg, D-His, D-Leu, D-Pro, D-Ser, D-Tyr, D-Val, D-allo-Thr, D-Asp, D-Lys, D-Trp, D-allo-Ile, D-Asn, D-Phe, D-Ala, D-Gln, D-Glu, D-Cys, D-Met, and D-Thr as an active ingredient. Such amino acids can exhibit moisture retainability (retention of or increase in the horny layer moisture content) as horny layer function improvement. The D-amino acid is preferably at least one D-amino acid selected from the group consisting of D-Arg, D-His, D-Leu, D-Tyr, D-Val, D-allo-Thr, D-Lys, D-Trp, D-allo-Ile, D-Asn, D-Phe, D-Gln, and D-Thr, more preferably at least one D-amino acid selected from the group consisting of D-His, D-Leu, D-Lys, D-Trp, D-allo-Ile, D-Asn, and D-Phe, and even more preferably at least one D-amino acid selected from the group consisting of D-His, D-Leu, D-Lys, D-Trp, D-allo-Ile, and D-Phe.

The D-amino acid is preferably at least one selected from the group consisting of D-Arg, D-His, D-Leu, D-Pro, D-Ser, D-Val, D-allo-Thr, D-Asp, D-Lys, D-Trp, D-allo-Ile, D-Asn, D-Phe, D-Ala, D-Gln, D-Glu, D-Cys, D-Met, and D-Thr and more preferably at least one selected from the group consisting of D-Arg, D-His, D-Leu, D-Val, D-allo-Thr, D-Lys, D-Trp, D-allo-Ile, D-Asn, D-Phe, D-Gln, and D-Thr. Such amino acids can exhibit barrier function (inhibition of the transepidermal water loss) as horny layer function improvement. Among these D-amino acids, the D-amino acid is preferably at least one selected from the group consisting of D-His, D-Leu, D-Lys, D-Trp, D-allo-Ile, D-Asn, and D-Phe and more preferably at least one selected from the group consisting of D-His, D-Leu, D-Lys, D-Trp, D-allo-Ile, and D-Phe, because Examples demonstrate that they promote the expression of a moisture retainability-related protein gene or the production of the protein (preferably filaggrin).

The D-amino acid is also preferably at least one selected from the group consisting of D-His, D-Leu, D-Pro, D-Tyr, D-Asp, D-Lys, D-Trp, D-allo-Ile, D-Asn, D-Phe, D-Ala, D-Gln, D-Glu, D-Cys, D-Met, and D-Thr and more preferably at least one selected from the group consisting of D-His, D-Leu, D-Tyr, D-Lys, D-Trp, D-allo-Ile, D-Asn, D-Phe, D-Gln, and D-Thr. Such amino acids can exhibit tiny wrinkle preventing or improving ability (a reduction in the wrinkle area rate) as horny layer function improvement. Among these D-amino acids, at least one selected from the group consisting of D-Leu, D-Lys, D-Trp, and D-Asn is even more preferred, and at least one selected from the group consisting of D-Leu, D-Lys, and D-Trp is still even more preferred, because Examples demonstrate that they promote the expression of the moisture retainability-related protein gene or the production of the protein (preferably filaggrin).

The inventors of the present invention have found out that there is a correlation between the content of the D-amino acid, which is contained only in an infinitesimal amount in living bodies, and the moisture retainability, the barrier function, and the tiny wrinkle preventing or improving ability of the skin and have studied the gene expression amount of proteins related to moisture retainability, barrier function, and tiny wrinkle preventing or improving ability and the expression amount of filaggrin using the D-amino acid for which the correlation has been found to find significant increases in the gene expression amount of the proteins and the expression amount of filaggrin.

Consequently, a new knowledge has been found out that the D-amino acid, which is contained only in an infinitesimal amount in living bodies, among amino acids in which the amino acids as racemic bodies themselves have been conventionally regarded as active ingredients has a strong influence on the production of the proteins related to the moisture retainability, the barrier function, and the tiny wrinkle preventing or improving ability of the skin and acts as an active ingredient. The horny layer function improving agent of the present invention, based on such a new knowledge, uses a marked effect of the D-amino acid, which is contained only in an infinitesimal amount in living bodies.

D-Cys may be low in stability like L-Cys (Japanese Patent Application Laid-open No. 2009-227660). For D-Asp and D-Glu, it may be required to adjust pH to be higher in order to improve solubility.

The amino acid has a D isomer and an L isomer as optical isomers. Among these, natural proteins contain L-amino acids that are peptide-bonded. It is known that limonene has optical isomers that differ in physiological activity, for example. However, the influence of the D-amino acid on proteins containing L-amino acids is currently under study, and no systematic understanding has been established.

The D-amino acid may be a salt thereof. The salt may be any salt pharmaceutically allowed. Examples thereof include alkaline metal salts such as a potassium salt and a sodium salt; alkaline earth metal salts such as a calcium salt, a barium salt, and a magnesium salt; ammonium salts such as an ammonium salt and a tricyclohexylammonium salt; alkanol amine salts such as a monoethanolamine salt, a diethanolamine salt, a triethanolamine salt, a monoisopropyl alcohol amine salt, a diisopropyl alcohol amine salt, and a triisopropyl alcohol amine salt.

The method for producing the D-amino acid is not limited to a particular method. Examples thereof include a synthesis method, an extraction method, an enzyme method, and a fermentation method. Another example thereof is a method that optically resolves a racemic mixture of D,L-amino acids. The optical resolution is not limited to a particular method and may be performed by a conventionally known method (e.g., a method that prepares a diastereomer salt using an optically resolving agent such as camphorsulfonic acid and performs resolution using solubility difference).

The horny layer function improvement is preferably at least any one of retention of the horny layer moisture content, inhibition of the transepidermal water loss, and a reduction in the wrinkle area rate. The retention of the horny layer moisture content can be evaluated by increasing a Corneometer measured value or a Skicon measured value, for example. The inhibition of the transepidermal water loss can be evaluated by decreasing a TEWL value, for example. The wrinkle area rate can be evaluated by producing a replica of the outer corner of the eye and measuring the wrinkle area rate with an instrument, for example.

### [1-1. Moisture Retainability Improving Agent]

A moisture retainability improving agent of the present invention contains at least one D-amino acid selected from the group consisting of D-Arg, D-His, D-Leu, D-Pro, D-Ser, D-Tyr, D-Val, D-allo-Thr, D-Asp, D-Lys, D-Trp, D-allo-Ile, D-Asn, D-Phe, D-Ala, D-Gln, D-Glu, D-Cys, D-Met, and D-Thr as an active ingredient. The D-amino acid is preferably at least one selected from the group consisting of D-Arg, D-His, D-Leu, D-Tyr, D-Val, D-allo-Thr, D-Lys, D-Trp, D-allo-Ile, D-Asn, D-Phe, D-Gln, and D-Thr. Among these D-amino acids, the D-amino acid is preferably at least one selected from the group consisting of D-His, D-Leu, D-Asp, D-Lys, D-Trp, D-allo-Ile, D-Asn, D-Phe, D-Ala, D-Glu, D-Cys, D-Met, D-Thr, and D-Pro, more preferably at least one selected from the group consisting of D-His, D-Leu, D-Lys, D-Trp, D-allo-Ile, D-Asn, and D-Phe, and even more preferably at least one selected from the group consisting of D-His, D-Leu, D-Lys, D-Trp, D-allo-Ile, and D-Phe, because Examples demonstrate that they promote the expression of the moisture retainability-related protein gene or the production of the protein (preferably filaggrin). For D-Arg, D-Ser, D-Tyr, D-Val, D-allo-Thr, and D-Gln, Examples do not demonstrate that they promote the production of the moisture retainability-related protein (preferably filaggrin). However, there is a correlation between the content of these D-amino acids and the moisture retainability of skin, and they are sufficiently presumed to be active ingredients improving moisture retainability through another mechanism of action.

The moisture retainability improving agent of the present invention may contain any moisture retainability improving component other than the above D-amino acids as needed.

The D-amino acids may be salts thereof, in which the salts of the D-amino acids are similar to those described above.

The moisture retainability improving agent of the present invention produces an effect of retaining or increasing the horny layer moisture content to improve moisture retainability. It is known that filaggrin moves from a granular layer to the horny layer, binds to keratin fiber and aggregates, forms a keratin pattern, and is then decomposed into a low molecular weight compound such as urocanic acid to be a natural moisturizing factor related to water retention and ultraviolet ray absorption. The natural moisturizing factor binds to water within cells to retain it, and a larger amount of the natural moisturizing factor can increase the horny layer moisture content. Consequently, it is considered that if the production of filaggrin can be promoted, the natural moisturizing factor as the decomposed product thereof is also produced in a large amount, which improves the moisture retainability of the skin.

### [1-2. Barrier function Improving Agent]

A barrier function improving agent of the present invention contains at least one D-amino acid selected from the group consisting of D-Arg, D-His, D-Leu, D-Pro, D-Ser, D-Val, D-allo-Thr, D-Asp, D-Lys, D-Trp, D-allo-Ile, D-Asn, D-Phe, D-Ala, D-Glu, D-Cys, D-Met, D-Gln, and D-Thr as an active ingredient. The D-amino acid is preferably at least one selected from the group consisting of D-Arg, D-His, D-Leu, D-Val, D-allo-Thr, D-Lys, D-Trp, D-allo-Ile, D-Asn, D-Phe, D-Thr, and D-Gln. Among these D-amino acids, the D-amino acid is preferably at least one selected from the group consisting of D-Asp, D-Lys, D-Trp, D-allo-Ile, D-Asn, D-Phe, D-Ala, D-His, D-Leu, D-Glu, D-Cys, D-Met, D-Thr, and D-Pro, more preferably at least one selected from the group consisting of D-Lys, D-Trp, D-allo-Ile, D-Asn, D-Phe, D-His, and D-Leu, and even more preferably at least one selected from the group consisting of D-His, D-Leu, D-Lys, D-Trp, D-allo-Ile, and D-Phe, because Examples demonstrate that they promote the production of barrier function-related proteins (filaggrin, involucrin, transglutaminase 1, keratin 10, and loricrin).

For D-Arg, D-Ser, D-Val, D-allo-Thr, and D-Gln, Examples do not demonstrate that they promote the production of the barrier function-related proteins (filaggrin, involucrin, transglutaminase 1, keratin 10, and loricrin). However, there is a correlation between the content of these D-amino acids and the barrier function of the skin, and they are sufficiently presumed to be active ingredients improving barrier function through another mechanism of action.

The barrier function improving agent of the present invention may contain any barrier function improving component other than the above D-amino acids as needed.

The D-amino acids may be salts thereof, in which the salts of the D-amino acids are similar to those described above.

The barrier function improving agent of the present invention produces an effect of improving barrier function that inhibits the entry of foreign substances such as viruses and bacteria from the outside. It is considered that filaggrin, involucrin, transglutaminase 1, keratin 10, and loricrin are the main causes of forming corneocytes and form a robust horny layer to prevent gaps from occurring in the horny layer.

### [1-3. Tiny Wrinkle Preventing or Improving Agent]

A tiny wrinkle preventing or improving agent of the present invention contains at least one selected from the group consisting of D-His, D-Leu, D-Pro, D-Tyr, D-Asp, D-Lys, D-Trp, D-allo-Ile, D-Asn, D-Phe, D-Ala, D-Glu, D-Gln, D-Cys, D-Met, D-Thr, D-Pro, and D-Glu as an active ingredient. The amino acid is preferably at least one selected from the group consisting of D-Leu, D-Tyr, D-Lys, D-Trp, D-allo-Ile, D-Asn, D-Phe, D-His, D-Gln, and D-Thr. Among these D-amino acids, preferred is at least one selected from the group consisting of D-Asp, D-Lys, D-Trp, D-allo-Ile, D-Asn, D-Phe, D-Ala, D-Glu, D-His, D-Pro, D-Cys, D-Met, D-Thr, and D-Leu, more preferred is at least one selected from the group consisting of D-Lys, D-Trp, D-Asn, and D-Leu, and even more preferred is at least one selected from the group consisting of D-Lys, D-Trp, and D-Leu, because the Examples demonstrate that they promote the production of filaggrin. For D-Tyr and D-Gln, Examples do not demonstrate that they promote the production of filaggrin. However, there is a correlation between the content of these D-amino acids and the wrinkle area rate, and they are sufficiently presumed to be active ingredients preventing or improving tiny wrinkles through another mechanism of action.

The tiny wrinkle preventing or improving agent of the present invention may contain any tiny wrinkle preventing or improving component other than the above D-amino acids as needed.

The D-amino acids may be salts thereof, in which the salts of the D-amino acids are similar to those described above.

The tiny wrinkle preventing or improving agent of the present invention produces an effect of making it possible of preventing or improving tiny wrinkles. It is said that the formation of wrinkles as one of the representative aging changes of the skin begins with tiny wrinkle formation caused by dryness of the skin, and improvement in moisture retention is considered to be a measure therefor. As described above, it is known that filaggrin moves from the granular layer to the horny layer, binds to keratin fiber and aggregates, forms a keratin pattern, and is then decomposed into a low molecular weight compound such as urocanic acid to be a natural moisturizing factor related to water retention and ultraviolet ray absorption. The natural moisturizing factor binds to water within cells to retain it, and a larger amount of the natural moisturizing factor can increase the horny layer moisture content. Consequently, if the production of filaggrin can be promoted, the natural moisturizing factor as the decomposed product thereof is also produced in a large amount, which improves the moisture retainability of the skin, and consequently, tiny wrinkle formation caused by dryness of the skin can also be prevented or improved.

### [1-4. Expression Promoter of Horny Layer Function-Related Protein Gene and Moisture Retention-Related Protein Production Promoter]

An expression promoter of a horny layer function-related protein gene of the present invention contains at least one selected from the group consisting of D-Asp, D-Lys, D-Trp, D-allo-Ile, D-Asn, D-Phe, D-Ala, and D-Glu and preferably at least one selected from the group consisting of D-Lys, D-Trp, D-allo-Ile, D-Asn, and D-Phe as an active ingredient.

A horny layer function-related protein (e.g., filaggrin) production promoter of the present invention preferably contains at least one D-amino acid selected from the group consisting of D-His, D-Leu, D-Pro, D-Asp, D-Lys, D-Trp, D-Allo-Ile, D-Phe, D-Ala, D-Glu, D-Cys, D-Met, and D-Thr as an active ingredient and more preferably contains at least one D-amino acid selected from the group consisting of D-His, D-Leu, D-Lys, D-Trp, D-allo-Ile, D-Phe, and D-Thr as an active ingredient.

In the present specification, the horny layer function-related protein is at least one selected from filaggrin, involucrin, loricrin, keratin 10, and transglutaminase 1.

Filaggrin is a protein strongly related to moisture retainability and is a source of supply of an amino acid as a natural moisturizing factor. Consequently, it is also a protein related to tiny wrinkle formation caused by dryness of the skin. In addition, it causes keratin filament to aggregate and to construct robust keratin and is thus a protein related also to barrier function.

Involucrin is a protein related to barrier function. It is one of the proteins forming the horny layer and is essential to the formation of a robust horny layer.

Keratin 10 is a protein related to barrier function. It is the main component of the horny layer.

Loricrin is a protein related to barrier function. It is one of the proteins forming the horny layer and is essential to the formation of a robust horny layer.

Transglutaminase 1 is an enzyme that cross-links proteins. It is considered to be important for the formation of a robust horny layer.

### [2. Skin Cosmetic and Skin External Preparation]

A skin cosmetic or skin external preparation of the present invention contains at least one of the above agents. The content of the agent of the present invention may be adjusted as appropriate in accordance with the type of the skin cosmetic and skin external preparation. A preferred lower limit of the content in terms of the total amount of the D-amino acid is normally 0.01% by mass or more, preferably 0.1% by mass or more, and more preferably 1% by mass or more. The upper limit thereof is normally 30% by mass or less and preferably 10% by mass or less.

The skin cosmetic and skin external preparation of the present invention may further contain at least one base selected from the group consisting of a polyol compound, a surfactant, a higher alcohol, a thickener, a chelating agent, an preservative, and a silicone and preferably contains it.

### (Polyol Compound)

Examples of the polyol compound include ethylene glycol, butylene glycol, polyethylene glycol, butylethyl propanediol, a polypropylene glycol copolymer, propylene glycol, dipropylene glycol, 1,3-butylene glycol, glycerin, diglycerin, and sorbitol. Among them, preferred is at least one selected from the group consisting of butylene glycol, glycerin, polyethylene glycol, dipropylene glycol, and sorbitol.

### (Surfactant)

Examples of the surfactant include anionic surfactants such as polyoxyethylene sorbitan oleate, higher fatty acid soaps, alkyl sulfates, polyoxyethylene alkyl ether sulfates, alkyl ether phosphates, N-acylamino acid salts, and acyl N-methyl taurine salts; cationic surfactants such as alkyltrimethylammonium chloride and dialkyldimethylammonium chloride; amphoteric surfactants such as cocamidopropyl betaine, alkyldimethylaminoacetic acid betaine, alkylamidodimethylaminoacetic acid betaine, and 2-alkyl-N-carboxy-N-hydroxyimidazolinium betaine; and nonionic surfactants such as glycerin fatty acid esters, polyglycerin fatty acid esters, lecithin derivatives (e.g., lysolecithin), polyoxyethylene type ones (e.g., polyoxyethylene hydrogenated castor oil, polyoxyethylene alkyl ethers, polyoxyethylene polyoxypropylene alkyl ethers, and polyoxyethylene glycerin fatty acid esters), polyhydric alcohol ester type ones, and an ethylene oxide/propylene oxide block copolymer. Among them, preferred is at least one selected from the group consisting of glycerin fatty acid esters, polyglycerin fatty acid esters, lecithin derivatives, polyoxyethylene hydrogenated castor oil, polyoxyethylene alkyl ethers, polyoxyethylene polyoxypropylene alkyl ethers, and polyoxyethylene glycerin fatty acid esters.

### (Higher Alcohol)

Examples of the higher alcohol include cetanol, behenyl alcohol, and isohexadecyl alcohol. Among them, behenyl alcohol is preferred.

### (Thickener)

Examples of the thickener include polyacrylic acid, carrageenan, xanthan gum, sodium carboxymethyl cellulose, carboxyvinyl polymer, polyoxyethylene glycol distearate, an acrylic acid-based polymer, a cellulose-based natural polymer, ethanol, neopentyl glycol dicaprate, and potassium myristate. Among them, preferred is at least one selected from the group consisting of an acrylic acid-based polymer, xanthan gum, and a cellulose-based natural polymer.

### (Chelating Agent)

Examples of the chelating agent include etidronic acid and ethylenediaminetetraacetic acid (derivatives) such as trisodium edetate. Among them, ethylenediaminetetraacetic acid is preferred.

### (Preservative)

For the preservative, at least either paraben or phenoxy ethanol is preferred. Examples of paraben include methylparaben, propylparaben, and butylparaben.

### (Silicone)

Examples of the silicone include methylpolysiloxane, dimethylpolysiloxane, methylphenylpolysiloxane, decamethylcyclopentasiloxane, cyclohexasiloxane, cyclopentasiloxane, trimethylsiloxysilicate, a polyoxyethylene/methylpolysiloxane copolymer, silicone fluid, silicone rubber, and silicone oil.

The skin cosmetic or skin external preparation of the present invention may contain one or two or more bases other than the above. The other bases may be bases used for normal skin cosmetics or skin external preparations; examples thereof include oil-based bases, water-soluble bases, emulsifiers, stabilizers, pH regulators, preservatives, ultraviolet ray absorbing agents, ultraviolet ray scattering agents, other functional components (physiologically active components), perfumes such as triethylhexanoin, pigments, solvents, natural product extracts, and amino acids (derivatives) other than the above D-amino acids.

Examples of oil-based bases include vegetable oils such as castor oil, cottonseed oil, sesame oil, jojoba oil, olive oil, and cacao butter; waxes such as Japan wax, lanolins such as liquid lanolin, candelilla wax, beeswax, and carnauba wax; higher hydrocarbons such as paraffins such as Vaseline, liquid paraffin, and solid paraffin, squalane, olefin oligomers, and plastibase; fatty acids such as lauryl acid, myristic acid, stearic acid, and palmitic acid and esters thereof; base waxes such as kerosene; and natural polymers and hydrogenated polyisobutene.

Examples of water-soluble bases include polyvinyl alcohol, cellulose derivatives (e.g., methylcellulose, hydroxyethyl cellulose, hydroxypropyl cellulose, and cationized cellulose), carboxyvinyl polymer, and esters (e.g., isopropyl myristate, isopropyl palmitate, stearyl stearate, octyldodecyl myristate, octyldodecyl oleate, and triglyceride 2-ethylhexanoate).

Examples of emulsifiers include stearyl alcohol, glyceryl monostearate, sorbitan monopalmitate, polyoxyethylene cetyl ether, polyoxyethylene sorbitan monolaurate, polyoxyethylene sorbitan monooleate, diglycerin monostearate, polysorbate 60, and phytosteryl/decyltetradecyl myristoyl methyl-β-alanine.

Examples of stabilizers include spherical alkyl polyacrylate powder, dimethyldistearylammonium hectorite, ascorbic acid, sodium pyrosulfite, gellan gum, and pectin.

Examples of pH regulators include a phosphate buffer, sodium hydroxide, potassium hydroxide, and triethanolamine.

Examples of preservatives include ethyl para-oxybenzoate, sodium benzoate, salicylic acid, sorbic acid, sodium hydrogen sulfite, and phenoxy ethanol.

Examples of ultraviolet ray absorbing agents include butyl methoxybenzoylmethane, octyl p-dimethylaminobenzoate, cinnamic acid derivatives such as 2-ethylhexyl p-methoxycinnamate, and ethylhexyl dimethoxybenzylidene dioxoimidazolidine propionate.

Examples of ultraviolet ray scattering agents include titanium oxide and zinc oxide.

Examples of solvents include an aqueous arginine solution and purified water.

Examples of pigments include Red 202, Yellow 4, Blue 1, Bengala, yellow iron oxide, and black iron oxide.

Examples of other functional components include kojic acid, trioleyl phosphate, squalane, cetyl ethylhexanoate, tocopherol, mica, dimer dilinoleyl bis(N-lauroyl-L-glutamate/N-lauroyl sarcosinate), tocopherol acetate, thiotaurine, honey, isopropyl lauroyl sarcosinate, lauroyl lysine, talc, dibutyl ethylhexanoyl glutamide, dibutyl lauroyl glutamide, bentonite, taurine, arbutin, panthenol, niacinamide, pyridoxine hydrochloride, retinol, carotene, riboflavin, ubiquinone, sodium hyaluronate, water-soluble collagen, sodium chondroitin sulfate, lactobacillus, milk ferment filtrate, whey, yogurt extract, sodium hydrolyzed casein, soybean ferment extract, benzalkonium chloride, triclosan, salicylic acid, citric acid, bisabolol, calcium chloride, glyceryl caprylate, mineral oil, sodium lauroyl glutamate, cocamide MEA, cocamide methyl MEA, polyvinylpyrrolidone, sodium cocoamphoacetate, polyquaternium, and guar hydroxypropyltrimonium chloride.

Examples of natural product extracts include lavender extract, peppermint extract, sage extract, anise extract, rose extract, Chamaecyparis obtusa water, rooibos extract, lavender extract, and Sophora angustifolia root extract.

Examples of amino acids (derivatives) include L-leucine, L-isoleucine, L-valine, L-phenylalanine, L-tryptophan, L-aspartic acid, sodium L-glutamate, L-lysine (hydrochloride), L-arginine, L-threonine, L-alanine, L-proline, L-serine, glycine, acetylglutamine, L-histidine, pyridoxylserine, carnosine, acetylmethionine, acetylcysteine, sodium polyaspartate, citrulline, ornithine, betaine, L-tyrosine, L-asparagine, and L-methionine.

The content of the other bases, which may be set as appropriate, is not limited to a particular content.

The dosage form of the skin cosmetic or skin external preparation of the present invention is not limited to a particular form; examples thereof include liquid, lotion, ointment, cream, plaster, tape formulation, powder, skin lotion, and gel. Among them, examples thereof include milky lotion, cream, skin lotion, and gel, and any of these is preferred.

The use site of the skin cosmetic or skin external preparation of the present invention is not limited to a particular site; it is normally applied to the skin and the mucosae. Among them, it is preferably used for the skin. Given this, examples of the use form of the skin cosmetic or skin external preparation of the present invention include aqueous solution, emulsion, powder dispersion, and emulsion (oil-in-water type, water-in-oil type, and the like). More specifically, examples thereof include liquid, oil, lotion, gel, sol, milky lotion, suspension, cream, ointment, patch, and stick. Examples of what is called cosmetics include skin lotions such as lotions and beauty liquids; milky lotions such as emollient milky lotions, milky lotions, nourishing milky lotions, and cleansing milky lotions; creams such as emollient creams, massage creams, cleansing creams, and makeup creams; sprays; cosmetics for makeup such as packs, foundations, rouges, lipsticks, eye shadows, blushers, white powders, and color powders and cosmetics for skin washing such as face cleansing agents, makeup removers, body shampoos, and soaps. The skin cosmetic or skin external preparation of the present invention may be used as quasi-drugs, drugs, and foods (including supplements and drinks) apart from the cosmetics.

Examples of the formulation of the skin cosmetic or skin external preparation of the present invention as a milky lotion or a cream include a formulation containing the agent of the present invention, a polyol group (at least one selected from the group consisting of butylene glycol, glycerin, polyethylene glycol, and dipropylene glycol), a surfactant group (at least one selected from the group consisting of glycerin fatty acid esters, polyglycerin fatty acid esters, and lecithin derivatives), a higher alcohol group (behenyl alcohol), a thickener group (at least either an acrylic acid-based polymer or xanthan gum), a chelating agent group (ethylenediaminetetraacetic acid), an preservative group (at least either paraben or phenoxy ethanol), and a silicone.

Examples of the formulation of the skin cosmetic or skin external preparation of the present invention as a skin lotion include a formulation containing the agent of the present invention, a polyol group (at least one selected from the group consisting of butylene glycol, glycerin, polyethylene glycol, and sorbitol), a surfactant group (at least one selected from the group consisting of polyoxyethylene hydrogenated castor oil, polyoxyethylene alkyl ethers, polyoxyethylene polyoxypropylene alkyl ethers, and polyoxyethylene glycerin fatty acid esters), a thickener group (at least one selected from the group consisting of an acrylic acid-based polymer, xanthan gum, and a cellulose-based natural polymer), a chelating agent group (ethylenediaminetetraacetic acid), an preservative group (at least either paraben or phenoxy ethanol), and a silicone.

Examples of the formulation of the skin cosmetic or skin external preparation of the present invention as a gel include a formulation containing the agent of the present invention, a polyol group (at least one selected from the group consisting of butylene glycol, glycerin, and polyethylene glycol), a surfactant group (at least one selected from the group consisting of polyoxyethylene hydrogenated castor oil, polyoxyethylene alkyl ethers, polyoxyethylene polyoxypropylene alkyl ethers, and polyoxyethylene glycerin fatty acid esters), a thickener group (at least one selected from the group consisting of an acrylic acid-based polymer, xanthan gum, and a cellulose-based natural polymer), a chelating agent group (ethylenediaminetetraacetic acid), an preservative group (at least either paraben or phenoxy ethanol), and a silicone.

The moisture retainability improving agent of the present invention can be expected to be used for prevention, inhibition, and treatment of rough skin, tiny wrinkles, itching, and the like caused by aging, dryness, skin diseases (e.g., atopic dermatitis), and the like through moisture retaining action that retains or increases the horny layer moisture content.

The barrier function improving agent of the present invention can be expected to be used for prevention, inhibition, and treatment of rough skin, inflammation, sensitive skin, and the like caused by aging, dryness, skin diseases (e.g., atopic dermatitis), and the like through barrier function improving action that inhibits the entry of foreign substances such as viruses and bacteria from the outside.

The tiny wrinkle preventing or improving agent of the present invention can be expected to be used for prevention, inhibition, and treatment of tiny wrinkles caused by dryness in particular through tiny wrinkle preventing or improving action that reduces the wrinkle area rate.

Consequently, the horny layer function improving agent, the gene expression promoter, and the skin cosmetic or skin external preparation of the present invention can be expected to be used for prevention, inhibition, and treatment of rough skin, tiny wrinkles, itching, inflammation, sensitive skin, and the like caused by aging, dryness, skin diseases (e.g., atopic dermatitis), and the like through the moisture retaining action that retains or increases the horny layer moisture content, the barrier function improving action that inhibits the entry of foreign substances such as viruses and bacteria from the outside, and the tiny wrinkle preventing or improving action.

### [3. Method of Evaluation]

A method of evaluation of the present invention is a method of evaluation of a skin by a D-amino acid including measuring at least one selected from the group consisting of a horny layer moisture content, a wrinkle area rate, and a transepidermal water loss of a skin of a subject to determine at least one selected from the group consisting of moisture retainability, tiny wrinkle preventing or improving ability, and barrier function of the skin of the subject, collecting a horny layer from a measurement site of the skin of the subject, identifying at least one D-amino acid level contained in the horny layer, and comparing a relation between the at least one selected from the group consisting of moisture retainability, barrier function, and tiny wrinkle preventing or improving ability of the skin and the D-amino acid level among results of the subject.

The method of evaluation of the present invention broadly includes the following two embodiments. One embodiment is an embodiment that compares a relation between the moisture retainability, the barrier function, and the tiny wrinkle preventing or improving ability of the skin and each component among results of a subject (a spare subject) (which may be a comparison among a plurality of results of the same subject or a comparison among results of a plurality of different subjects) and, when a correlation is determined, selects a correlation between the appearance of the skin and the D-amino acid level as an indicator of an evaluation of the skin of the subject (which may be the same as or different from the spare subject). Another embodiment is an embodiment that identifies a D-amino acid level in the horny layer before and after a subject is dosed with a sample of a skin cosmetic, a skin external preparation, a candidate substance thereof, or the like and compares evaluations of the skin before and after dosing obtained in light of the above correlation with each other to perform an evaluation of the sample or an evaluation of efficacy to the skin of the subject.

The subject may be a man or a woman; however, the subject is preferably a woman because of sensitiveness to care of skin conditions and is more preferably a woman in her twenties to thirties who begins to have interest in care of skin conditions by aging or a woman in her forties to sixties who often take care of skin conditions by aging.

In the method of evaluation of the present invention, the moisture retainability of the skin is normally evaluated by the horny layer moisture content. The barrier function of the skin is normally evaluated by the transepidermal water loss (TEWL). The tiny wrinkle preventing or improving ability of the skin is normally evaluated by the wrinkle area rate.

The horny layer present on the outermost layer of epidermis plays a role of a barrier that separates an external environment and the inside of the body. The quality of the horny layer is the main cause of determining the freshness of the skin. The horny layer contains components such as an intercellular lipid and a natural moisturizing factor (NMF); in addition to water evaporation from within the skin, the horny layer itself retains proper water, thereby retaining softness and/or smoothness of the surface of the skin. The horny layer moisture content and TEWL are used as indicators evaluating the quality of the horny layer.

Examples of the method for measuring the horny layer moisture content as a moisture content present in the horny layer of the skin include a method that measures the electric impedance of the skin using a high-frequency current, a method of measurement using instruments such as Skicon-200EX (manufactured by Yayoi Co., Ltd.) that passes a high-frequency current of 3.5 MHz through the skin via electrodes to measure conductance and Corneometer (manufactured by Courage+Khazaka Germany) that estimates a moisture content from capacitance, a method using infrared spectra, a method using microwaves, and a photoacoustic method. Among them, preferred is a method that measures conductance on the surface of the skin. It can be said that a larger horny layer moisture content indicates more excellent moisture retainability.

Examples of the method for measuring TEWL include a method that brings the skin into contact with one end of a cylindrical probe with both ends open to measure a moisture content evaporated from the surface of the skin using a humidity-and-temperature sensor installed within the probe. Tewameter (registered trademark) TM300 (manufactured by Courage+Khazaka) is a measurement apparatus employing the method. It can be said that a smaller TEWL value indicates more excellent barrier function.

Examples of the method for measuring the wrinkle area rate include the following method. A replica of the outer corner of the eye is produced using SKIN CAST (manufactured by RSI), this replica is measured using Primos lite 1813 (manufactured by GFMesstechnik GmbH), and the wrinkle area rate can be calculated by the accompanying software. It can be said that a smaller wrinkle area rate indicates more excellent tiny wrinkle preventing or improving ability.

In the method of evaluation of the present invention, the method for determining the various kinds of functions of the skin is not limited to the above ones; they can be determined by other conventionally known methods using indicators such as the number of days of horny layer turnover and a horny layer nuclear cell rate, for example.

The measurement site of the skin is not limited to a particular site; however, it is preferably a site exposed to the outside in daily lives in view of evaluating the horny layer playing a role of a barrier that separates an external environment and the inside of the body. Examples thereof include a cheek and an arm. The measurement site may be changed in accordance with the method of measurement. When the horny layer moisture content or the TEWL value is measured, a cheek can be measured, whereas when the wrinkle area rate is measured, the outer corner of the eye can be measured, for example.

The measurement site may be one or two or more.

The horny layer may be collected from a certain measurement site of the skin by a conventionally known method such as tape stripping. Tape stripping is a method that puts adhesive tape on the skin and peels off the adhesive tape to collect a horny layer attached to an adhesive face.

Examples of the method for collecting the horny layer include a method that, when commercially available adhesive tape is pressed against a measurement site and is then removed therefrom, collects a horny layer attached to the adhesive layer. Examples of the commercially available adhesive tape include Horny Layer Checker manufactured by Asahi Biomed Co., Ltd., Horny Layer Sticker manufactured by Moritex Corporation, Horny Layer Checker (Disc Type W, Disc Type G, and PRO Type) manufactured by Promotool Corporation, Corneofix manufactured by Integral Corporation, Transparent Tape manufactured by 3M, Transparent Double-Sided Tape manufactured by 3M, and Cellotape (registered trademark) manufactured by Nichiban Co., Ltd.

The method for identifying the D-amino acid level is not limited to a particular method; examples thereof include a direct method (a method that performs precolumn derivatization and then performs separation by chromatography with a chiral stationary phase column) and an indirect method (a method that performs derivatization to a diastereomer with a chiral derivatizing reagent and then performs separation by chromatography with a reversed phase column).

Examples of the direct method include a method that performs precolumn fluorescence derivatization with NBD-F, then performs separation with a chiral column, and performs fluorescence detection. The details thereof can be referred to "Comprehensive analysis of branched aliphatic D-amino acids in mammals using an integrated multi-loop two-dimensional column-switching high-performance liquid chromatographic system combining reversed-phase and enantioselective columns." J Chromatogr A. 2007 Mar 2; 1143(1-2): 105-11. Hamase K, Morikawa A, Ohgusu T, Lindner W, Zaitsu K.

Examples of the indirect method include a method that performs derivatization with OPA and chiral thiol, performs separation with a reversed phase column, and performs fluorescence detection and a method that performs derivatization with DBD-PyNCS, performs separation with a reversed phase column, and performs detection with a mass spectrometer. The details thereof can be referred to "High-performance liquid chromatography analysis of naturally occurring D-amino acids in sake." J Chromatogr B Analyt Technol Biomed Life Sci. 2011 Nov 1;879(29):3259-67. Gogami Y, Okada K, Oikawa T. "Determination of DL-amino acids, derivatized with R(-)-4-(3-isothiocyanatopyrrolidin-1-yl)-7-(N,N-dimethylaminosulfonyl)-2,1,3-benz oxadiazole, in nail of diabetic patients by UPLC-ESI-TOF-MS" J. Chromatogr. B, 879, 3220-3228 (2011). J. Z. Min, S. Hatanaka, H.F. Yu, T. Higashi, S. Inagaki, T. Toyo'oka.

The component of the D-amino acid to be identified may be one or a combination of two or more and preferably contains D-Arg, D-His, D-Leu, D-Pro, D-Ser, D-Tyr, D-Val, D-allo-Thr, D-Asp, D-Lys, D-Trp, D-allo-Ile, D-Asn, D-Phe, D-Ala, D-Gln, D-Glu, and D-Met.

It can be determined from the method of correlation evaluation of the present invention that the more preferred D-amino acids are components having a correlation with at least any one of the moisture retainability, the barrier function, and the tiny wrinkle preventing or improving ability of the skin of women of at least any age of twenties to thirties and forties to sixties.

The one embodiment of the method of evaluation of the present invention provides a new knowledge about a correlation between a specific D-amino acid and the moisture retainability, the barrier function, and the tiny wrinkle preventing or improving ability of the skin. Consequently, the method of evaluation of the present invention can be applied to development of skin cosmetics or skin external preparations of components derived from natural products or the like.

More specifically, the following can be said about the age of the subject and the type of the D-amino acid. When the subject is a woman in her twenties to thirties, the component of the D-amino acid is preferably at least one selected from the group consisting of D-Arg, D-His, D-Leu, D-Pro, D-Ser, D-Tyr, D-Val, D-allo-Thr, D-Asp, D-Lys, D-Trp, D-allo-Ile, D-Asn, and D-Phe. With this D-amino acid, at least moisture retainability can be evaluated.

When the subject is a woman in her twenties to thirties, the component of the D-amino acid is preferably at least one selected from the group consisting of D-His, D-Leu, D-Pro, D-Ser, D-Val, D-allo-Thr, D-Asp, D-Trp, D-allo-Ile, D-Asn, and D-Phe. With this D-amino acid, at least barrier function can be evaluated.

When the subject is a woman in her forties to sixties, the component of the D-amino acid is preferably at least one selected from the group consisting of D-Arg, D-His, D-Leu, D-Pro, D-Val, D-allo-Thr, D-Asp, D-Lys, D-Trp, D-allo-Ile, D-Asn, and D-Gln. With this D-amino acid, at least moisture retainability can be evaluated.

When the subject is a woman in her forties to sixties, the component of the D-amino acid is preferably at least one selected from the group consisting of D-Arg, D-His, D-Leu, D-Pro, D-Ser, D-Val, D-allo-Thr, D-Lys, D-Trp, D-allo-Ile, D-Phe, and D-Gln. With this D-amino acid, at least barrier function can be evaluated.

When the subject is a woman in her twenties to thirties, the component of the D-amino acid is preferably at least one selected from the group consisting of D-Leu, D-Tyr, D-Asp, D-Lys, D-Trp, D-Asn, and D-Gln. With this D-amino acid, at least tiny wrinkle preventing or improving ability can be evaluated.

When the subject is a woman in her forties to sixties, the component of the D-amino acid is preferably at least either D-Asp or D-Lys. With this D-amino acid, at least tiny wrinkle preventing or improving ability can be evaluated.

The method for identifying a D-amino acid level is not limited to a particular method; examples thereof include mass spectroscopy using liquid chromatography (LC) or gas chromatography (GC), an enzyme method using an enzyme for quantification, and bioassays.

Among those described above, a liquid chromatograph-tandem mass spectrometer is preferred, because identification of the D-amino acid and quantification of the content thereof can be simultaneously performed.

The D-amino acid level is preferably a D-amino acid content per content of protein contained in the horny layer. With this D-amino acid level, experimental errors derived from the method for collecting the horny layer can be reduced.

The method for measuring the content of protein is not limited to a particular method; examples thereof include methods of measurement using a spectrophotometer such as an ultraviolet ray absorption method, the BCA method, the Bradford method, the Lowry method, and the Biuret method and a method of measurement by electrophoresis. Among them, the BCA method is preferred.

The method of evaluation of the present invention obtains a relation between at least any one of the horny layer moisture content, TEWL, and the wrinkle area rate and the D-amino acid level from a plurality of subjects, compares them with each other, and can thereby identify a D-amino acid that correlates with at least any one of the moisture retainability, the barrier function, and the tiny wrinkle preventing or improving ability of the skin. The method of comparison is not limited to a particular method; examples thereof include a method that plots measured values of the horny layer moisture content, TEWL, and the wrinkle area rate and the D-amino acid level on a graph.

By using the method of evaluation of the present invention, the moisture retainability, the barrier function, and the tiny wrinkle preventing or improving ability of the skin can be evaluated by the content of the D-amino acid. Consequently, before and after the same subject is dosed with a sample of a skin cosmetic, a skin external preparation, or the like, the contents of the D-amino acid for which the correlation has been recognized are compared with each other, whereby the efficacy of the sample to the subject can be determined. Consequently, the method of evaluation of the present invention can also be used as a method that determines whether the skin cosmetic or skin external preparation is appropriate for the subject.

The other embodiment of the method of evaluation of the present invention can provide not only a short-term evaluation by the horny layer moisture content, TEWL, or the wrinkle area rate like conventional methods of measurement but also an evaluation about whether skin conditions are improved in a long term by the content of the D-amino acid before and after dosing of the skin cosmetic or skin external preparation.

### [Examples]

The following describes the present invention in detail with reference to Examples; the Examples do no limit the present invention.

### (Measurements of Horny Layer Moisture Content, TEWL, and Wrinkle Area Rate)

Subjects (all were women; age structure: twenties to thirties: 31, forties to sixties: 40; a total number of 71) washed their faces and were adapted in a constant-temperature constant-humidity room (humidity: 40%, temperature: 20°C) for 20 minutes.

After adaptation, a horny layer moisture content was measured at the cheek central part of the subjects using Skicon-200EX (manufactured by Yayoi Co., Ltd.). A TEWL value was measured at the cheek central part of the subjects using Tewameter (registered trademark) TM300 (manufactured by Courage+Khazaka Germany).

After the measurements of the horny layer moisture content and the TEWL value, a replica of the outer corner of the eye was produced using SKIN CAST (manufactured by RSI). This replica was then measured using Primos lite 1813 (manufactured by GFMesstechnik GmbH), and a wrinkle area rate was calculated by the accompanying software.

### (Horny Layer Collection by Tape Stripping)

After the end of the measurements, a piece of tape cut into 3 cm × 3 cm was attached to the cheek central part of the subjects to collect a horny layer. A horny layer on the outermost layer collected for the first time was not used for analysis, and a horny layer collected for the second or third time was used for analysis.

### (D-Amino Acid Extraction Operation)

The collected tape was put into a conical tube (50 ml), was hermetically sealed, and was stored at -80°C. The collected tape (horny layer) was subjected to ultrasonic treatment twice using a 95% aqueous methyl alcohol solution, and a water-soluble component was extracted. To the extracted liquid, an internal standard liquid (containing a plurality of D,L-amino acid stable isotopes such as D-Arg) was added. The obtained aqueous methyl alcohol solution was dried and solidified with a centrifugal evaporator to be a sample for D-amino acid analysis.

### (Quantitative Analysis of D-Amino Acid)

Water in an amount of 50 µL was added to the concentrated and dried and solidified sample, which was stirred with a vortex mixer to be redissolved to be a sample solution. A borate buffer solution with a concentration of 200 mM (pH 8.8) in an amount of 10 µL was added to 20 µL of the sample solution, which was stirred, and then 20 µL of a 5 mg/mL (R)-N-(hydroxysuccinimidyl carbamate)-β-(3-pyridyl)-alanine methyl ester/acetonitrile solution was added thereto, and the solution was reacted at room temperature for 10 minutes. A 0.1 M aqueous hydrochloric acid solution in an amount of 60 µL was added to the reaction solution to be a sample for analysis. The analysis was performed under the following conditions using

### LC/MS/MS.

### (HPLC Conditions)

Apparatus: Nexera X2 (manufactured by Shimadzu Corporation)
Guard column: Waters ACQUITY BEH C18 VanGuard (2.1 × 5 mm, 1.7 µm)
Column: Waters ACQUITY UPLC BEH C18 (2.1 × 100 mm, 1.7 µm)
Mobile Phase A: 10 mM aqueous ammonium bicarbonate solution (pH 9.5)
Mobile Phase B: 80% methanol/water
Flow rate: 0.5 mL/min
Column temperature: 50°C
Sample injection amount: 1 µL

Table 1 shows a gradient condition.

**[Table 1]**

| Time(min) | B(%) |
|---|---|
| 0.1 | 2 |
| 4 | 8 |
| 6 | 13.5 |
| 11 | 30 |
| 14 | 32 |
| 16 | 37 |
| 17 | 80 |
| 18 | 80 |
| 18.1 | 2 |
| 20 | 2 |

### (Mass Analysis Conditions)

Apparatus: Triple Quad 6500 (SCIEX)
Method of Ionization: ESI, Positive mode
Scan type: SRM (Selected Reaction Monitoring)

Table 2 shows a transition.

**[Table 2]**

| amino acids | Q1 | Q3 |
|---|---|---|
| D,L-Ala | 296.0 | 207.0 |
| D,L-Ser | 312.0 | 207.0 |
| D,L-Asp | 340.1 | 207.0 |
| D,L-Asn | 339.1 | 207.1 |
| D,L-Trp | 411.2 | 207.1 |
| D,L-Gln | 353.1 | 207.2 |
| D,L-Phe | 372.2 | 207.1 |
| D,L-Glu | 354.1 | 207.2 |
| D,L-Pro | 322.1 | 207.0 |
| D,L-Arg | 381.2 | 201.1 |
| D,L-Val | 324.1 | 207.1 |
| D,L-Leu | 338.1 | 207.1 |
| D-allo-Ile,,L-Ile | 338.1 | 207.1 |
| D-allo-Thr,,L-Thr | 326.1 | 207.0 |
| D,L-His | 362.1 | 181.1 |
| D,L-Lys | 559.2 | 181.0 |
| D,L-Met | 356.1 | 207.2 |
| D,L-Tyr | 594.2 | 388.2 |
| Gly | 282.1 | 207.0 |

### (Protein Extraction)

The tape after the water-soluble component was extracted was put into a microtube, and an insoluble fraction was eluted using hexane. After the tape was removed, the insoluble fraction was washed with hexane to be a sample for protein quantitative analysis.

### (Protein Quantitative Analysis)

An aqueous NaOH (0.5 mol/L)-SLS (0.05 wt%) solution was added to a tube used in "D-Amino Acid Extraction Operation" or the like and was heated for 60 minutes at 90°C. A protein eluted in these solutions was quantified.

For the quantification of the protein, the MicroBCA method was used; the same amount of a sample solution and a MicroBCA solution were added and were shaken while being heated at 50°C (1,100 rpm) for 1 hour. Absorbance (562 nm) was measured with a spectrometer to quantify the protein.

### (Examples and Comparative Examples of Method of Evaluation)

Table 3 lists correlations between the contents of the D-amino acids in the horny layer of the subjects and the horny layer moisture content, the TEWL value, and the wrinkle area rate. Cases with an absolute value of a correlation coefficient of 0.4 or more were evaluated to be "A" as having "a strong correlation", cases with that of 0.3 or more were evaluated to be "B" as having "a medium correlation," and cases with that of 0.2 or more were evaluated to be "C" as having "a weak correlation."

**[Table 3]**

| | Moisture retainability | | Barrier function | | Tiny wrinkle preventing or improving ability | |
|---|---|---|---|---|---|---|
| | Horny layer moisture content | | TEWL | | Wrinkle area rate | |
| | Young woman | Elderly woman | Young woman | Elderly woman | Young woman | Elderly woman |
| D-Asn | A | B | A (FIG.13) | | B (FIG.16) | |
| D-Asp | A (FIG.10) | C | C | | C | C |
| D-Lys | B | C | | C | C | B (FIG.18) |
| D-Phe | A (FIG.11) | | A (FIG.14) | C | | |
| D-Trp | C | C | C | C | C | |
| D-allo-Ile | A | A (FIG.12) | B | B (FIG.15) | | |
| D-Arg | C | C | | C | | |
| D-His | B | C | B | C | | |
| D-Leu | A | C | A | C | C | |
| D-Pro | A | B | A | C | | |
| D-Ser | A | | A | B | | |
| D-Tyr | C | | | | C | |
| D-Val | A | A | B | B | | |
| D-allo-Thr | A | B | B | C | | |
| D-Gln | | C | | C | A (FIG.17) | |

As can be seen from Table 3, the contents of D-Asn, D-Asp, D-Lys, D-Trp, D-Leu, and D-Gln in the horny layer were recognized to have correlations with the horny layer moisture content, the TEWL value, and the wrinkle area rate for at least either young women or elderly women. The contents of D-Phe, D-allo-Ile, D-Arg, D-His, D-Pro, D-Ser, D-Val, and D-allo-Thr in the horny layer were recognized to have correlations with the horny layer moisture content and the TEWL value for at least either young women or elderly women. The content of D-Tyr in the horny layer was recognized to have correlations with the horny layer moisture content and the wrinkle area rate for young women.

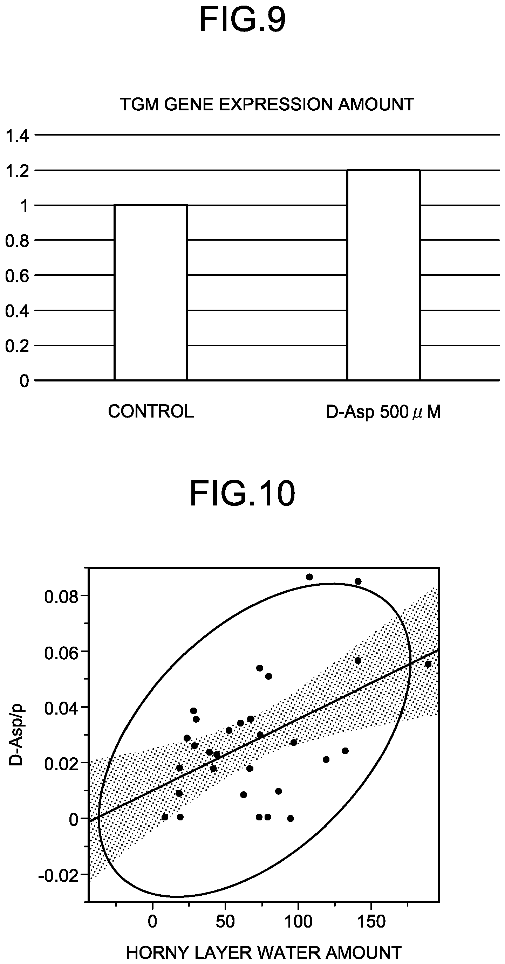

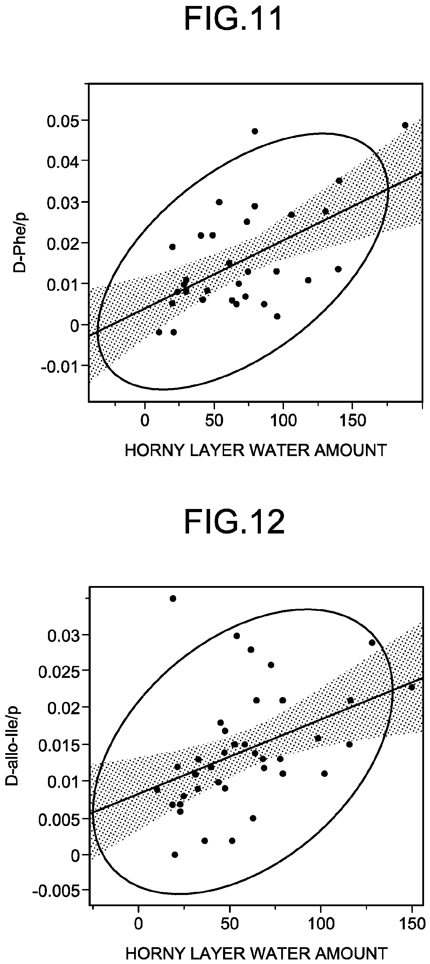

FIGs. 10 to 12 illustrate plots comparing the value of the horny layer moisture content and the content of the D-amino acid with each other. In FIGS. 10 to 12, the amino acid content is a value obtained by dividing the amino acid content determined in "Quantitative Analysis of D-Amino Acid" by the content of protein determined in "Protein Quantitative Analysis."

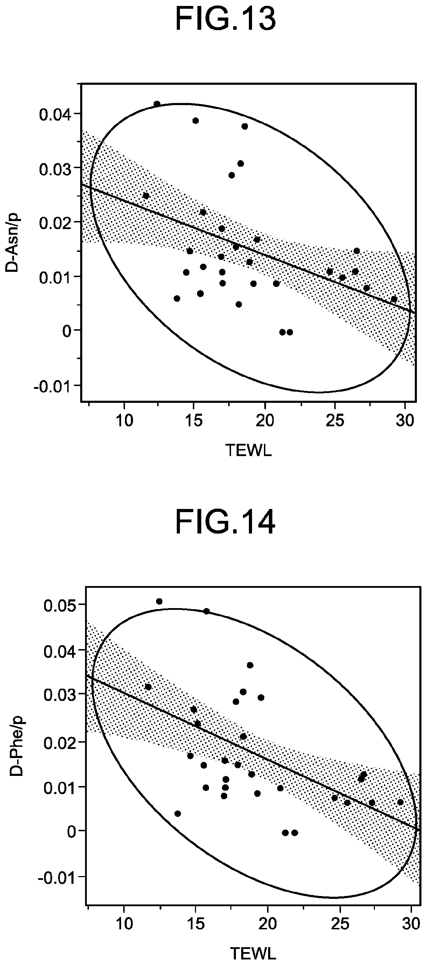

FIGS. 13 to 15 illustrate plots comparing the value of TEWL and the content of the D-amino acid with each other. In FIGS. 13 to 15, the amino acid content is a value obtained by dividing the amino acid content determined in "Quantitative Analysis of D-Amino Acid" by the content of protein determined in "Protein Quantitative Analysis."

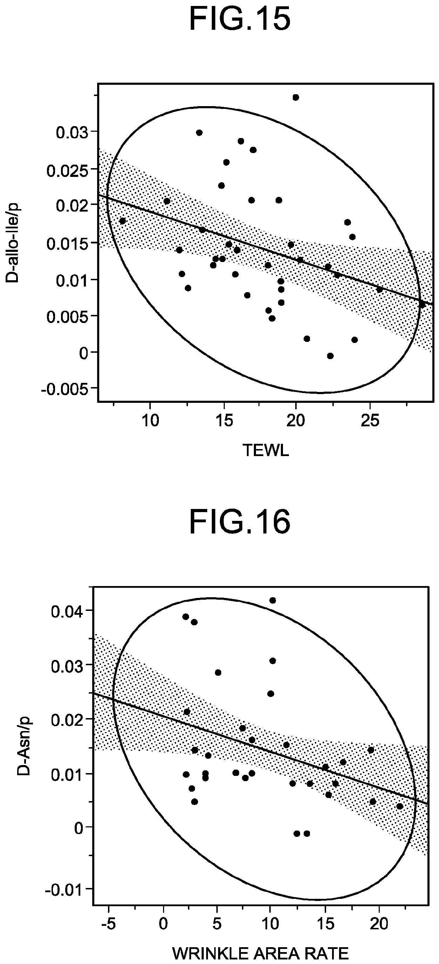

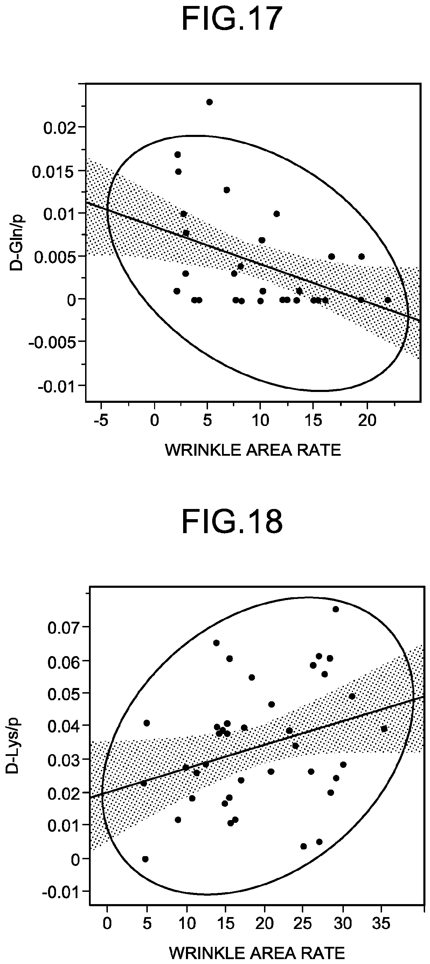

FIGS. 16 to 18 illustrate plots comparing the wrinkle area rate and the content of the D-amino acid with each other. In FIGS. 16 to 18, the amino acid content is a value obtained by dividing the amino acid content determined in "Quantitative Analysis of D-Amino Acid" by the content of protein determined in "Protein Quantitative Analysis."

### (Examples 1 to 8 of Horny Layer Function Improving Agent) (Gene Expression Test on "Moisture Retention/Barrier function/Tiny Wrinkle Preventing or Improving Ability" of D-Amino Acid Using Normal Human Epidermal Keratinocytes)

Normal human epidermal keratinocytes (Kurabo Industries Ltd.) were cultured with HuMedia KG2 (Kurabo Industries Ltd.) at 37°C and 5% CO₂ under saturated vapor. Cells that have become confluent were seeded in a 6-well plate with a seeding density of 3.0 × 10⁵ (cells/well).

For each evaluation sample, each D-amino acid adjusted in the culture medium so as to be 100 µM or 500 µM was used. On the next day of seeding, each evaluation sample was adjusted to be 100 µM or 500 µM, and each well culture medium was exchanged. Culturing was performed for 48 hours, RNA was extracted from the cells using RN easy mini kit (Qiagen), which was synthesized into cDNA using High-Capacity cDNA Reverse Transcription Kit with RNase Inhibitor (Thermo Fisher). With this cDNA as a template, a sample added group and a control group were compared with each other by the RT-PCR method (7500 fast system (Thermo Fisher)) to evaluate gene expression amounts of filaggrin, involucrin, loricrin, keratin 10, and transglutaminase 1. Table 4 lists results. In Table 4, FLG, IVL, K10, LOR, and TGM indicate filaggrin, involucrin, keratin 10, loricrin, and transglutaminase 1, respectively.

**[Table 4]**

| | | | FLG | IVL | K10 | LOR | TGM |
|---|---|---|---|---|---|---|---|
| Example 1 | D-Asn | 100 *µ*M | | | | | |
| | | 500 *µ*M | 1.55 | 1.34 | 1.21 | 1.80 | |
| Example 2 | D-Asp | 100 *µ*M | 1.35 | 1.49 | 2.43 | 2.34 | 1.13 |
| | | 500 *µ*M | 1.45 | 1.42 | 2.10 | 2.10 | 1.20 |
| Example 3 | D-Lys | 100 *µ*M | 1.48 | 1.27 | 1.35 | 2.02 | 1.12 |
| | | 500 *µ*M | 1.29 | 1.72 | 2.16 | 1.39 | |
| Example 4 | D-Phe | 100 *µ*M | | | | | |
| | | 500 *µ*M | 1.52 | 1.32 | 1.32 | 1.78 | 1.26 |
| Example 5 | D-Trp | 100 *µ*M | 1.77 | 2.05 | 2.44 | 1.71 | 1.42 |
| | | 500 *µ*M | 3.30 | 2.43 | 3.43 | 3.45 | 1.50 |
| Example 6 | D-allo-Ile | 100 *µ*M | 1.30 | 1.18 | 1.31 | 1.31 | |
| | | 500 *µ*M | 1.21 | 1.13 | 1.41 | | 1.27 |
| Example 7 | D-Ala | 100 *µ*M | 1.23 | 1.28 | 1.47 | | 1.20 |
| | | 500 *µ*M | 1.18 | 1.15 | 1.21 | | |
| Example 8 | D-Glu | 100 *µ*M | | | | | |
| | | 500 *µ*M | 1.69 | 1.34 | 1.25 | 2.07 | 1.13 |

As can be seen from Table 4, by using D-Asp, D-Lys, D-Phe, D-Trp, D-allo-Ile, and D-Glu, increases in the expression amounts of the respective target genes were recognized. By using D-Ala, increases in the expression amounts of the respective target genes of filaggrin, involucrin, keratin 10, and transglutaminase 1 were recognized. By using D-Asn, increases in the expression amounts of the respective target genes of filaggrin, involucrin, keratin 10, and loricrin were recognized.

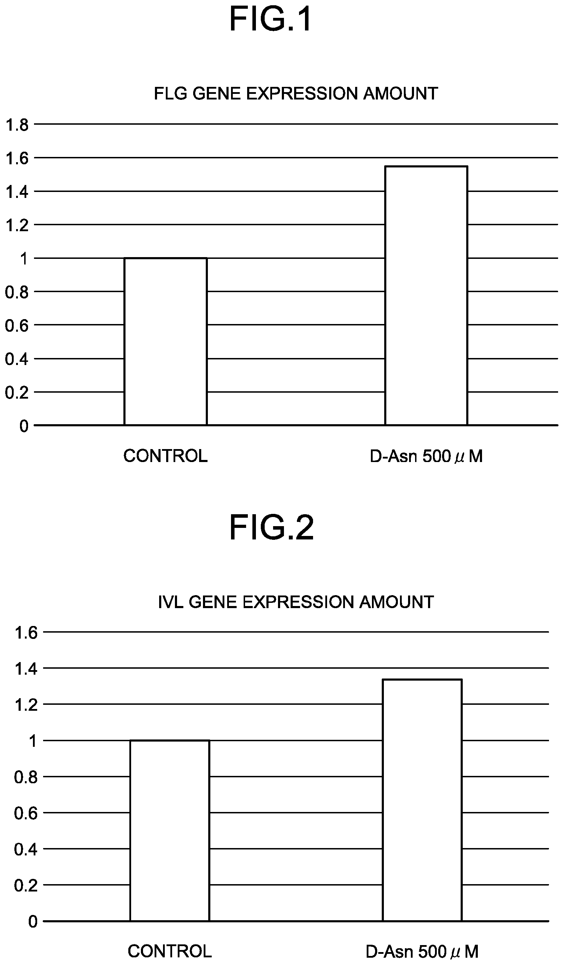

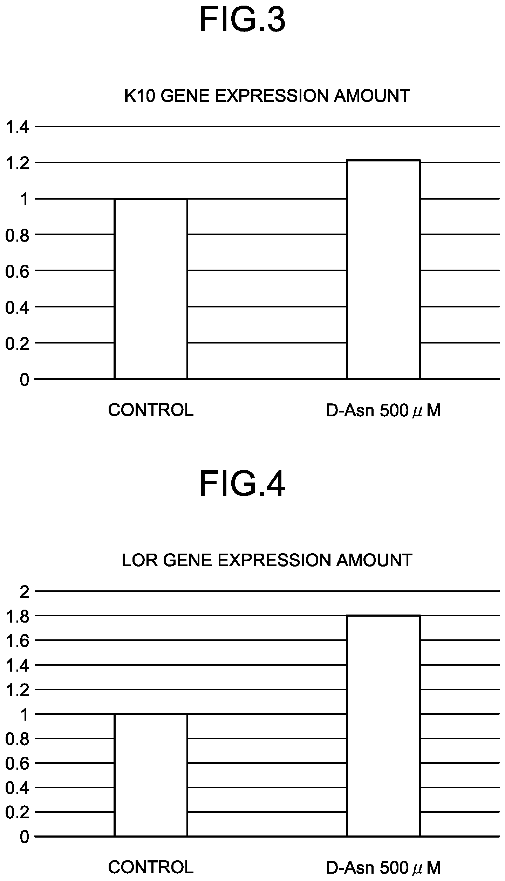

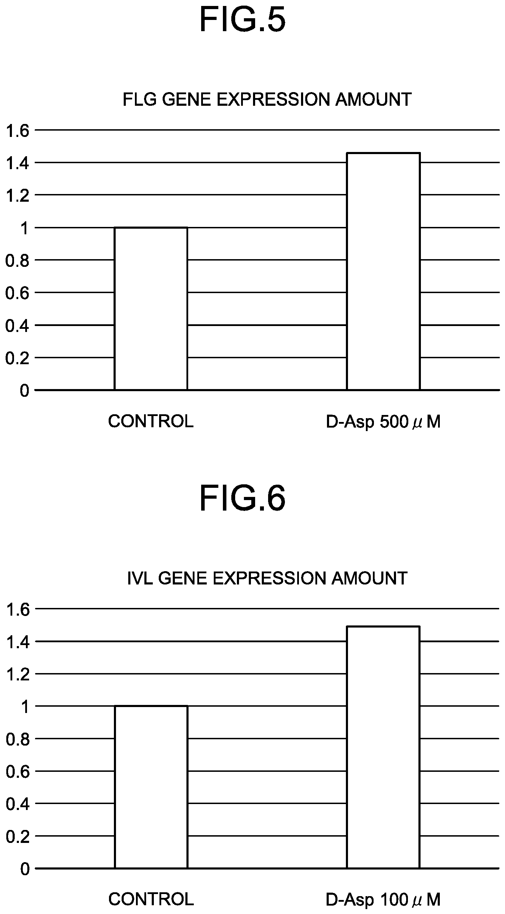

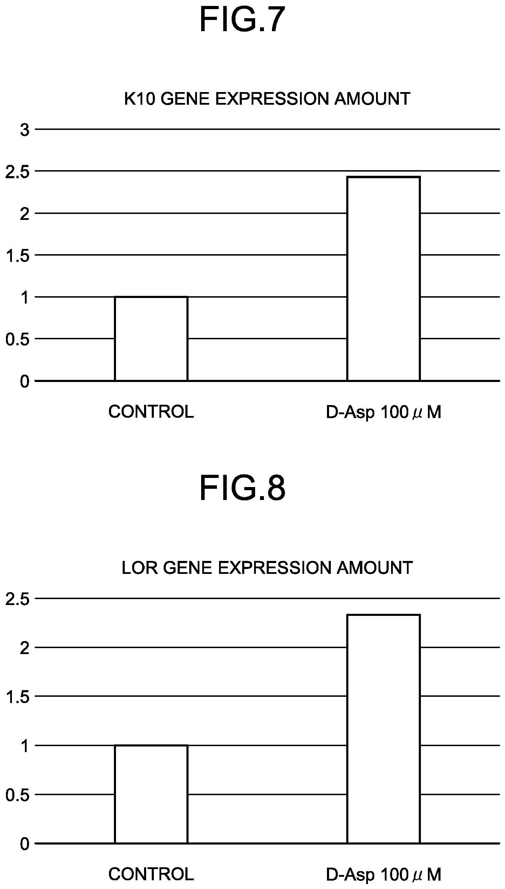

FIGS. 1 to 4 are graphs of increases (relative values to increases of controls) of filaggrin, involucrin, keratin 10, and loricrin when D-Asn (Example 1) is used. FIGS. 5 to 9 are graphs of increases (relative values to increases of controls) of filaggrin, involucrin, keratin 10, loricrin, and transglutaminase 1 when D-Asp (Example 2) is used (note that FIGS. 5 and 9 are 500 µM, whereas FIGS. 6 to 8 are 100 µM).

These results show that the D-amino acid promotes the production of the proteins contributing to moisture retainability, barrier function, and tiny wrinkle preventing or improving ability and is useful for an active ingredient of the horny layer function improving agent and the like.

### Example 9

### (Cytotoxicity Test: Neutral Red Method)

Normal human epidermal keratinocytes were seeded in a 6-well plate, and on the next day, a sample with a D-amino acid (500 µM) adjusted in a cell culturing culture medium was added thereto. After 48 hours, the evaluation sample solution was removed from the plate, and each well was rinsed using 2 ml of a cell culture medium. A culture medium with neutral red (NR) in it (Kurabo Industries Ltd.) was added dropwise with 2 ml/well and was allowed to stand for 2 hours at 37°C and 5% CO₂ under saturated vapor.

The culture medium (with neutral red in it) was discarded, a washing fixative (a 2 wt% calcium chloride solution and a 2 wt% formalin solution mixed with the equal amount) was added with 2 ml/well, and after being allowed to stand for 1 minute, the washing fixative was removed. An NR extract (50% by volume ethyl alcohol and 1% by volume acetic acid) was added with 2 ml/well and was shaken with a plate shaker for 15 minutes. Inclusion of NR into living cells was examined by measuring the absorbance at 540 nm of the NR extract with a microplate reader (manufactured by Thermo Fisher Scientific). The absorbance of the NR extract of each sample-added cell with a certain concentration when the measured value (absorbance) of the NR extract of a control (without sample addition) was defined as 100% was represented as relative percentage, whereby the cytotoxicity of each sample was calculated.

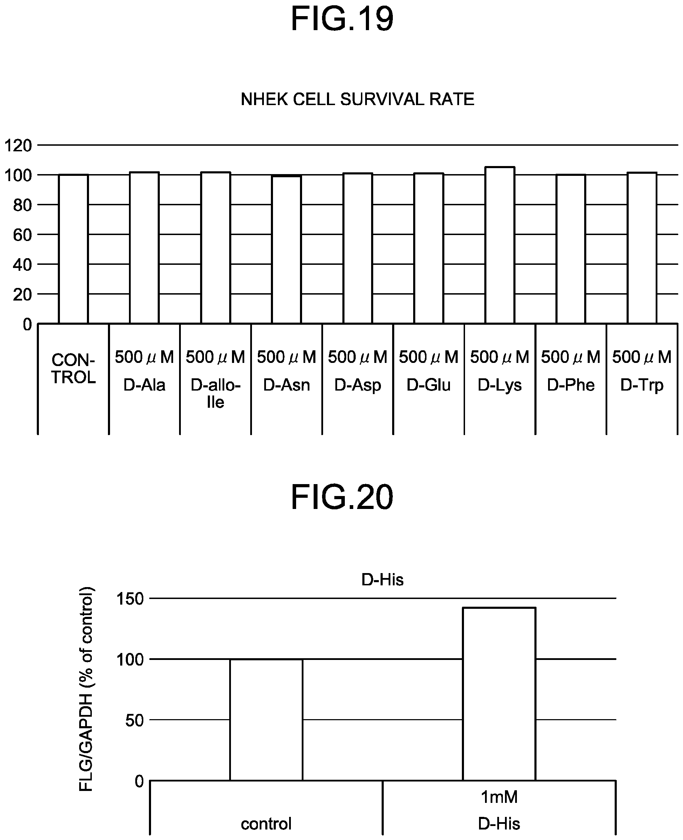

### FIG. 19 illustrates results.

As can be seen from FIG. 19, it was recognized that each evaluated D-amino acid was free of cytotoxicity in 500 µM. It has been found out that, in the concentration free of cytotoxicity, the D-amino acid induces increase and decrease in gene expression leading to various kinds of skin improvement effects such as moisture retainability, barrier function, and tiny wrinkle preventing or improving ability.

### Example 10

### (Filaggrin Production Promotion Test)

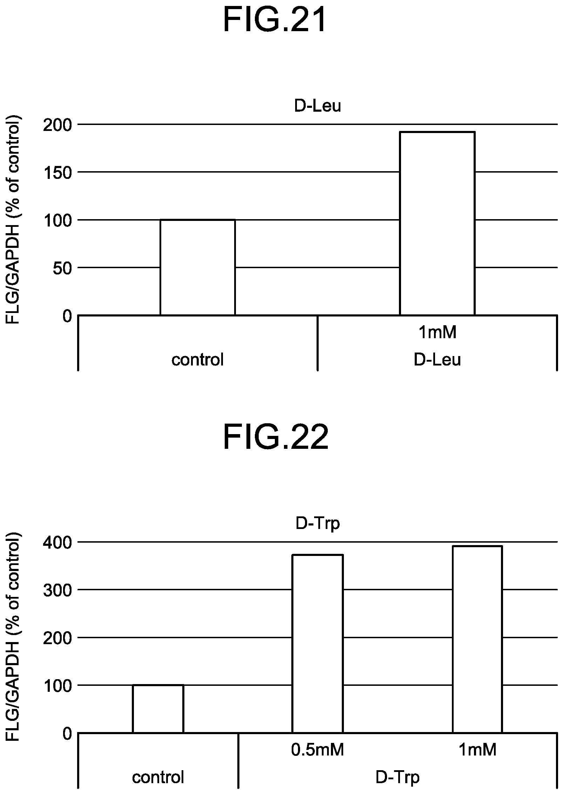

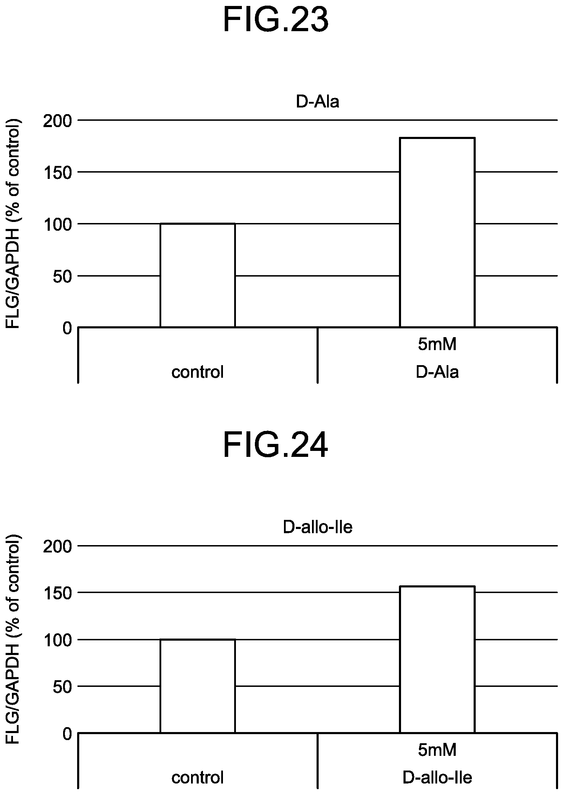

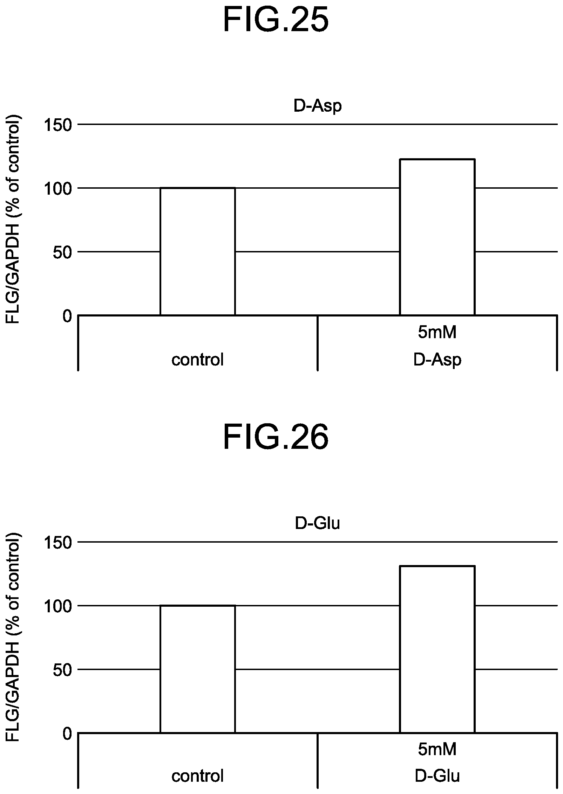

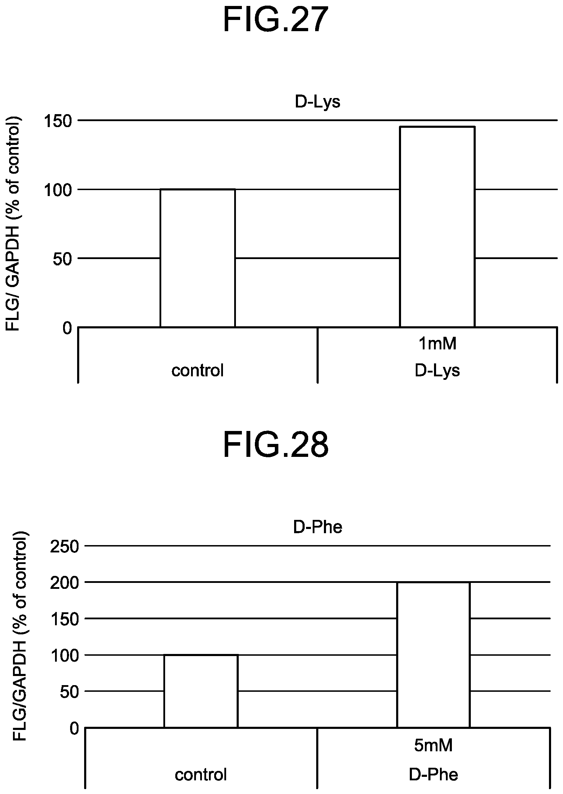

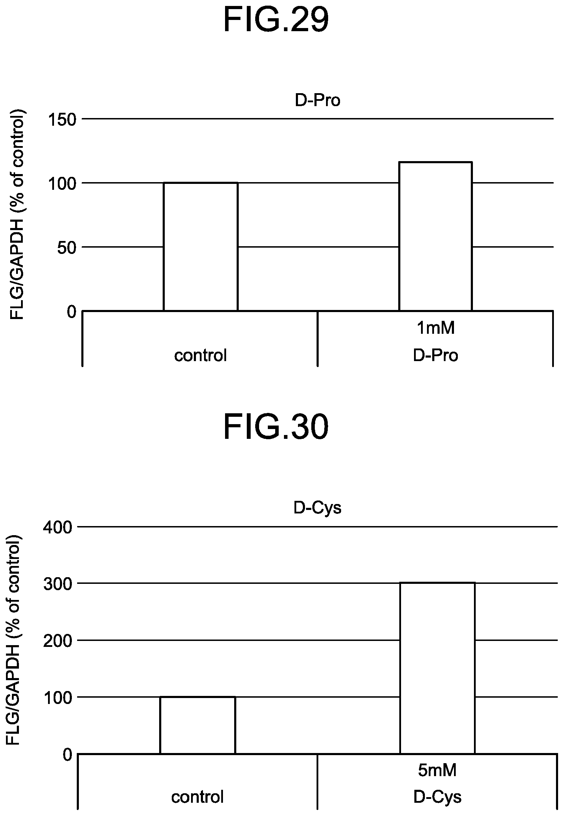

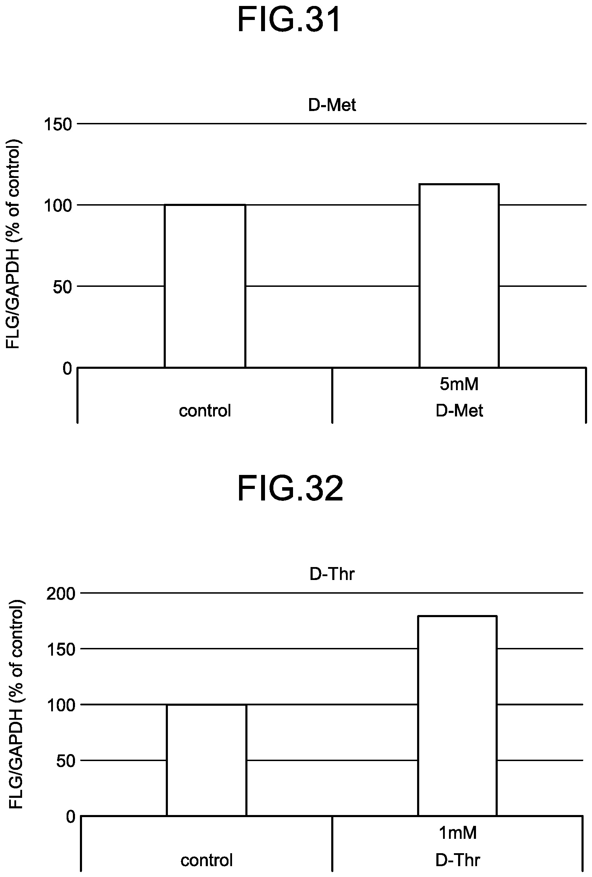

Normal human neonatal epidermal keratinocytes (Kurabo Industries Ltd.) were cultured with HuMedia KG2 (Kurabo Industries Ltd.). Cells that have become confluent was peeled off with trypsin and were seeded in a 6-well plate with 15 × 10⁴ (cells/well). On the next day, after the cells adhered to the plate, exchange with HuMedia KG2 with each evaluation sample (a control (without sample addition) and the D-amino acid) and calcium chloride added in certain evaluation concentrations (0.5 mM, 1 mM, or 5 mM for the sample and 1.3 mM for calcium chloride) was performed, which was cultured for 5 days. During culture, culture medium exchange was performed with HuMedia KG2 with the sample and the calcium chloride added once in two days or three days. After culture, the culture medium was removed and was washed twice with cool PBS (-), and an extract (M-PER (Thermo Fisher Scientific)) with protease inhibitor and phosphatase inhibitor (EzRIPA Lysis (Atto Corporation)) added was added to each well with 200 µL/well, and the plate was shaken with a multi shaker (As One Corporation) at 400 rpm for 5 minutes. The cells were scraped together with a scraper, and 200 µL of a denaturant (EzApply (Atto Corporation)) was added to the entire liquid including precipitate, which was then shaken with a shaker (Bioer Technology) while being heated at 100°C at 1,100 rpm for 5 minutes. The obtained solution was centrifuged with a centrifuge (Tomy Seiko Co., Ltd.) at 10,000 G at 4°C for 10 minutes, and the supernatant was used as a sample for Western blot. Per one lane of gel, 10 µL of each sample for Western blot (profilaggrin) or 5 µL of a solution obtained by diluting the sample for Western blot by 20 times (GAPDH) was applied to e-PAGEL 7.5% (manufactured by Atto Corporation) and was electrophoresed with an electrophoresis apparatus (Atto Corporation) with a constant current of 21 mA for 1 hour. The gel after electrophoresis was transferred to a PVDF film, was washed using TBS-T (Atto Corporation), and was blocked using EzBlock BSA (Atto Corporation). Next, an antigen-antibody reaction was carried out with a primary antibody solution (Anti Filaggrin (AKH1) antibody (Santa Cruz Biotechnology) and Anti GAPDH (6C5) antibody (Santa Cruz Biotechnology)). Labelling was performed with a secondary antibody (Anti-IgG (H+L), Mouse, Goat-poly, HRP (KPL)), and band intensity (250-400 kDa (profilaggrin) and 37 kDa (GAPDH)) was numerically expressed with an image analyzer (Amersham) by chemiluminescence. For the value of profilaggrin, one normalized with the value of GAPDH was used. The profilaggrin amount of each sample when the profilaggrin amount in the control was set to 100% was calculated as relative percentage. FIGS. 20 to 32 illustrate results.

From FIGS. 20 to 32, it can be seen that the filaggrin production amount of the cells improves by the addition of each sample.

### (Prescription Example 1; essence)

**[Table 5]**

| Name of component | Amount (parts by mass) | Name of component | Amount (parts by mass) |
|---|---|---|---|
| Lysolecithin | 0.50 | D-Phe | 0.50 |
| Polyoxyethylene sorbitan monooleate (2 E.O.) | 0.30 | D-Ala | 0.50 |
| Polyoxyethylene hydrogenated castor oil (40 E.O.) | 0.50 | D-Gln | 0.50 |
| Ethanol | 7.00 | D-Glu | 0.50 |
| Methylphenylpolysiloxane | 3.00 | Kojic acid | 1.00 |
| Perfume | 0.01 | Trioleyl phosphate | 7.00 |
| D-Arg | 0.50 | 1,3-Butylene glycol | 3.00 |
| D-His | 0.50 | Polyethylene glycol 1000 | 1.00 |
| D-Leu | 0.50 | Potassium hydroxide | 0.15 |
| D-Pro | 0.50 | Lavender extract | 0.01 |
| D-Ser | 0.50 | Peppermint extract | 0.01 |
| D-Tyr | 0.50 | Sage extract | 0.01 |
| D-Val | 0.50 | Anise extract | 0.01 |
| D-allo-Thr | 0.50 | Rose extract | 0.01 |
| D-Asp | 0.50 | Chamaecyparis obtusa water | 0.01 |
| D-Lys | 0.50 | Rooibos extract | 0.01 |
| D-Trp | 0.50 | Lavender extract | 0.10 |
| D-allo-Ile | 0.50 | 10% Aqueous Arg solution | Proper amount |
| D-Asn | 0.50 | Purified water | Remainder |

### (Prescription Example 2; cream)

**[Table 6]**

| Name of component | Amount (parts by mass) | Name of component | Amount (parts by mass) |
|---|---|---|---|
| Squalane | 8.00 | D-Pro | 0.01 |
| Cetyl ethylhexanoate | 3.00 | D-Ser | 0.01 |
| Cetanol | 2.80 | D-Tyr | 0.01 |
| Stearic acid | 2.40 | D-Val | 0.01 |
| PG stearate | 1.20 | D-allo-Thr | 0.01 |
| Glyceryl stearate (SE) | 3.30 | D-Asp | 0.01 |
| Polysorbate 60 | 0.50 | D-Lys | 0.01 |
| PEG-40 stearate | 1.50 | D-Trp | 0.01 |
| Tocopherol | 0.05 | D-allo-Ile | 0.01 |
| Phytosteryl/decyltetradecyl myristoyl methyl-β-alanine | 1.00 | D-Asn | 0.01 |
| | | D-Phe | 0.01 |
| Methylparaben | 0.20 | D-Ala | 0.01 |
| BG | 5.00 | D-Gln | 0.01 |
| Xanthan gum | 0.10 | D-Glu | 0.01 |
| Gellan gum | 0.10 | Lavender extract | 0.10 |
| D-Arg | 0.01 | 10% Aqueous L-Arg solution | Proper amount |
| D-His | 0.01 | Perfume | Proper amount |
| D-Leu | 0.01 | Purified water | Remainder |

### (Prescription Example 3; milky lotion)

**[Table 7]**

| Name of component | Amount (parts by mass) | Name of component | Amount (parts by mass) |
|---|---|---|---|
| Stearic acid | 2.00 | D-Ala | 0.50 |
| Cetanol | 1.00 | D-Gln | 0.50 |
| Liquid paraffin | 9.00 | D-Glu | 0.50 |
| Cetyl ethylhexanoate | 1.00 | L-Arg | 0.01 |
| Jojoba oil | 1.00 | L-His | 0.01 |
| Squalane | 2.00 | L-Leu | 0.01 |
| POE (30) hydrogenated castor oil | 3.00 | L-Pro | 0.01 |
| Methylphenylpolysiloxane | 1.00 | L-Ser | 0.01 |
| Tocopherol | 0.30 | L-Val | 0.01 |
| POE (10) monooleate | 0.10 | L-Thr | 0.01 |
| Butylparaben | 0.20 | L-Asp | 0.01 |
| PG | 5.00 | L-Lys hydrochloride | 0.01 |
| BG | 2.00 | L-Trp | 0.01 |
| Carboxyvinyl polymer | 0.50 | L-Ile | 0.01 |
| Kojic acid | 1.00 | L-Phe | 0.01 |
| D-Arg | 0.50 | L-Ala | 0.01 |
| D-His | 0.50 | Acetylglutamine | 0.01 |
| D-Leu | 0.50 | Sodium L-Glu | 0.01 |
| D-Pro | 0.50 | Gly | 0.01 |
| D-Ser | 0.50 | Pyridoxylserine | 0.01 |
| D-Tyr | 0.50 | Carnosine | 0.01 |
| D-Val | 0.50 | Acetylmethionine | 0.01 |
| D-allo-Thr | 0.50 | Acetylcysteine | 0.01 |
| D-Asp | 0.50 | Lavender extract | 0.10 |
| D-Lys | 0.50 | EDTA-2Na | 0.05 |
| D-Trp | 0.50 | Etidronic acid | 0.05 |
| D-allo-Ile | 0.50 | 10% Aqueous L-Arg solution | Proper amount |
| D-Asn | 0.50 | Perfume | Proper amount |
| D-Phe | 0.50 | Purified water | Remainder |

### (Prescription Example 4; rouge)

**[Table 8]**

| Name of component | Amount (parts by mass) | Name of component | Amount (parts by mass) |
|---|---|---|---|
| Candelilla wax | 8.00 | D-Pro | 0.20 |
| Paraffin | 6.00 | D-Ser | 0.20 |
| Beeswax | 3.00 | D-Tyr | 0.10 |
| Carnauba wax | 2.00 | D-Val | 0.10 |
| Lanolin | 7.17 | D-allo-Thr | 0.10 |
| Castor oil | 13.30 | D-Asp | 0.20 |
| Cetyl ethylhexanoate | 13.03 | D-Lys | 0.20 |
| Ethylhexyl palmitate | 10.32 | D-Trp | 0.20 |
| Hydrogenated polyisobutene | 5.00 | D-allo-Ile | 0.20 |
| Red 202 | 1.05 | D-Asn | 0.20 |
| Yellow 4 | 0.90 | D-Phe | 0.20 |
| Blue 1 | 0.04 | D-Ala | 0.20 |
| Titanium oxide | 1.60 | D-Gln | 0.10 |
| Diisostearyl malate | 3.59 | D-Glu | 0.20 |
| Mica | 2.00 | Dimer dilinoleyl bis(N-lauroyl-L-glutamate/N-lauroyl sarcosinate) | 20.00 |
| D-Arg | 0.20 | | |
| D-His | 0.20 | | |
| D-Leu | 0.20 | | |

### (Prescription Example 5; ointment)

**[Table 9]**

| Name of component | Amount (parts by mass) | Name of component | Amount (parts by mass) |
|---|---|---|---|
| Octyl p-dimethylaminobenzoate | 4.00 | D,L-Asp | 0.60 |
| Butyl methoxybenzoylmethane | 0.50 | D,L-Lys | 0.60 |
| Tocopherol acetate | 0.50 | D,L-Trp | 0.60 |
| Retinol palmitate | 1.00 | D,L-allo-Ile | 0.60 |
| Stearyl alcohol | 18.00 | D,L-Asn | 0.60 |
| Japan wax | 20.00 | D,L-Phe | 0.60 |
| D,L-Arg | 0.60 | D,L-Ala | 0.60 |
| D,L-His | 0.60 | D,L-Gln | 0.60 |
| D,L-Leu | 0.60 | D,L-Glu | 0.60 |
| D,L-Pro | 0.60 | Lavender extract | 0.10 |
| D,L-Ser | 0.60 | Polyoxyethylene (10) monooleate | 0.25 |
| D,L-Tyr | 0.60 | Glycerin monostearate | 0.30 |
| D,L-Val | 0.60 | | |
| D,L-allo-Thr | 0.60 | Vaseline | 45.15 |

### (Prescription Example 6; sunscreen)

**[Table 10]**

| Name of component | Amount (parts by mass) | Name of component | Amount (parts by mass) |
|---|---|---|---|
| Dimethylpolysiloxane | 5.00 | D-allo-Thr | 0.01 |
| Decamethylcyclopentasiloxane | 25.00 | D-Asp | 0.01 |
| Trimethyl siloxysilicate | 5.00 | D-Lys | 0.01 |
| Polyoxyethylene/methylpolysiloxane copolymer | 2.00 | D-Trp | 0.01 |
| 2-Ethylhexyl p-methoxycinnamate | 7.50 | D-allo-Ile | 0.01 |
| Spherical alkyl polyacrylate powder | 5.00 | D-Asn | 0.01 |
| Dextrin palmitate-coated fine particle zinc oxide (60 nm) | 5.00 | D-Phe | 0.01 |
| | | D-Ala | 0.01 |
| DPG | 5.00 | D-Gln | 0.01 |
| Dimethyldistearylammonium hectorite | 0.50 | D-Glu | 0.01 |
| | | Thiotaurine | 0.05 |
| D-Arg | 0.01 | Sophora angustifolia root extract | 1.00 |
| D-His | 0.01 | Lavender extract | 0.10 |
| D-Leu | 0.01 | Methylparaben | Proper amount |
| D-Pro | 0.01 | Phenoxy ethanol | Proper amount |
| D-Ser | 0.01 | Trisodium edetate | Proper amount |
| D-Tyr | 0.01 | Butylethyl propanediol | 0.50 |
| D-Val | 0.01 | Purified water | Remainder |
| | | Perfume | Proper amount |

### (Prescription Example 7; skin lotion)

**[Table 11]**

| Name of component | Amount (parts by mass) | Name of component | Amount (parts by mass) |
|---|---|---|---|
| Sorbitol | 4.00 | Acetylmethionine | 0.01 |
| DPG | 6.00 | Acetylcysteine | 0.01 |
| PEG-30 | 5.00 | D-Arg | 0.03 |
| POE (20) oleyl alcohol ether | 0.50 | D-His | 0.03 |
| | | D-Leu | 0.03 |
| Methylcellulose | 0.20 | D-Pro | 0.03 |
| Pyrus cydonia seed | 0.10 | D-Ser | 0.03 |
| Ethanol | 10.00 | D-Tyr | 0.03 |
| L-Arg | 0.01 | D-Val | 0.03 |
| L-His | 0.01 | D-allo-Thr | 0.03 |
| L-Leu | 0.01 | D-Asp | 0.03 |
| L-Pro | 0.01 | D-Lys | 0.03 |
| L-Ser | 0.01 | D-Trp | 0.03 |
| L-Val | 0.01 | D-allo-Ile | 0.03 |
| L-Thr | 0.01 | D-Asn | 0.03 |
| L-Asp | 0.01 | D-Phe | 0.03 |
| L-Lys hydrochloride | 0.01 | D-Ala | 0.03 |
| L-Trp | 0.01 | D-Gln | 0.03 |
| L-Ile | 0.01 | D-Glu | 0.03 |
| L-Phe | 0.01 | Lavender extract | 0.10 |
| L-Ala | 0.01 | EDTA-2Na | 0.05 |
| Acetylglutamine | 0.01 | Etidronic acid | 0.05 |
| Sodium L-Glu | 0.01 | Tocopherol | 0.30 |
| Gly | 0.01 | 10% Aqueous L-Arg solution | Proper amount |
| Pyridoxylserine | 0.01 | Perfume | Proper amount |
| Carnosine | 0.01 | Purified water | Remainder |

### (Prescription Example 8; jelly pack)

**[Table 12]**

| Name of component | Amount (parts by mass) | Name of component | Amount (parts by mass) |
|---|---|---|---|
| Glycerin | 10 | Betaine | 0.05 |
| Diglycerin | 3 | D-Amino acid | 8.5 |
| BG | 3 | Arbutin | 0.10 |
| DPG | 1 | Panthenol | 0.10 |
| PEG-30 | 0.5 | Niacinamide | 0.10 |
| Honey | 2 | Pyridoxine HCl | 0.10 |
| Xanthan gum | 0.1 | Retinol | 0.10 |
| Carrageenan | 0.5 | Carotene | 0.10 |
| Pectin | 0.5 | Riboflavin | 0.10 |
| Jojoba oil | 5 | Tocopherol | 0.10 |
| Neopentyl glycol dicaprate | 5 | Ubiquinone | 0.10 |
| Dimethicone | 0.1 | Sodium hyaluronate | 0.10 |
| Cyclohexasiloxane | 0.1 | Water-soluble collagen | 0.10 |
| Cyclopentasiloxane | 0.1 | Sodium chondroitin sulfate | 0.10 |
| Phytosteryl/decyltetradecyl myristoyl methyl-β-alanine | 0.2 | Lactobacillus/Milk ferment filtrate | 5.00 |
| Isopropyl lauroyl sarcosinate | 0.2 | Whey | 1.00 |
| Lauroyl lysine | 0.1 | Yogurt extract | 1.00 |
| Talc surface-treated with lauroyl lysine | 0.1 | Sodium hydrolyzed casein | 0.10 |
| Ethylhexyl dimethoxybenzylidene dioxoimidazolidine propionate | 0.1 | Soybean ferment extract | 0.10 |
| Dibutyl ethylhexanoyl glutamide | 0.1 | Lavender extract | 0.10 |
| Dibutyl lauroyl glutamide | 0.1 | Benzalkonium chloride | 0.10 |
| Bentonite | 0.1 | Triclosan | 0.10 |
| Polysorbate 65 | 2.5 | Salicylic acid | 0.10 |
| L-Amino acid | 1.35 | Citric acid | 0.10 |
| Acetylglutamine | 0.05 | Bisabolol | 0.10 |
| Acetylcysteine | 0.05 | Calcium chloride | 0.10 |
| Sodium polyaspartate | 0.05 | EDTA-2Na | 0.10 |
| Pyridoxylserine | 0.05 | Glyceryl caprylate | 0.10 |
| Taurine | 0.05 | Phenoxy ethanol | 0.30 |
| Carnosine | 0.05 | Ethanol | 0.50 |
| Citrulline | 0.05 | Perfume | Proper amount |
| Ornithine | 0.05 | Purified water | Remainder |

Table 13 lists the details of D-amino acid and L-amino acid in Table 12.

**[Table 13]**

| L-Amino acid | Amount (parts by mass) | D-Amino acid | Amount (parts by mass) |
|---|---|---|---|
| L-Arg | 0.50 | D-Arg | 0.50 |
| L-His | 0.05 | D-His | 0.50 |
| L-Leu | 0.05 | D-Leu | 0.50 |
| L-Pro | 0.05 | D-Pro | 0.50 |
| L-Ser | 0.05 | D-Ser | 0.50 |
| L-Tyr | 0.05 | D-Tyr | 0.50 |
| L-Val | 0.05 | D-Val | 0.50 |
| L-Thr | 0.05 | D-allo-Thr | 0.50 |
| L-Asp | 0.05 | D-Asp | 0.50 |
| L-Lys | 0.05 | D-Lys | 0.50 |
| L-Trp | 0.05 | D-Trp | 0.50 |
| L-Ile | 0.05 | D-allo-Ile | 0.50 |
| L-Asn | 0.05 | D-Asn | 0.50 |
| L-Phe | 0.05 | D-Phe | 0.50 |
| L-Ala | 0.05 | D-Ala | 0.50 |
| L-Glu | 0.05 | D-Gln | 0.50 |
| Gly | 0.05 | D-Glu | 0.50 |
| L-Met | 0.05 | | |

### (Prescription Example 9; o/w type foundation)

**[Table 14]**

| Name of component | Amount (parts by mass) | Name of component | Amount (parts by mass) |
|---|---|---|---|
| Talc | 3.00 | L-His | 0.01 |
| Titanium oxide | 5.00 | L-Pro | 0.01 |
| Bengala | 0.50 | L-Ser | 0.01 |
| Yellow iron oxide | 1.40 | L-Val | 0.01 |
| Black iron oxide | 0.10 | L-Thr | 0.01 |
| Bentonite | 0.50 | L-Asp | 0.01 |
| Polysorbate 60 | 0.90 | L-Lys hydrochloride | 0.01 |
| Triethanolamine | 1.00 | L-Trp | 0.01 |
| PG | 10.00 | L-Ile | 0.01 |
| Kojic acid | 1.00 | L-Phe | 0.01 |
| D-Arg | 0.02 | L-Ala | 0.01 |
| D-His | 0.02 | Acetylglutamine | 0.01 |
| D-Leu | 0.02 | Sodium L-Glu | 0.01 |
| D-Pro | 0.02 | Gly | 0.01 |
| D-Ser | 0.02 | Pyridoxylserine | 0.01 |
| D-Tyr | 0.02 | Carnosine | 0.01 |
| D-Val | 0.02 | Acetylmethionine | 0.01 |
| D-allo-Thr | 0.02 | Acetylcysteine | 0.01 |
| D-Asp | 0.02 | Lavender extract | 0.10 |
| D-Lys | 0.02 | Stearic acid | 2.20 |
| D-Trp | 0.02 | Isohexadecyl alcohol | 7.00 |
| D-allo-Ile | 0.02 | Glyceryl stearate | 2.00 |
| D-Asn | 0.02 | Liquid lanolin | 2.00 |
| D-Phe | 0.02 | Liquid paraffin | 8.00 |
| D-Ala | 0.02 | 10% Aqueous L-Arg solution | Proper amount |
| D-Gln | 0.02 | Methylparaben | 0.20 |
| D-Glu | 0.02 | Perfume | Proper amount |
| L-Arg | 0.01 | Purified water | Remainder |

### (Prescription Example 10; makeup cleansing)

**[Table 15]**

| Name of component | Amount (parts by mass) | Name of component | Amount (parts by mass) |
|---|---|---|---|
| Mineral oil | 38.00 | D-Val | 0.01 |
| Octyl palmitate | 20.00 | D-allo-Thr | 0.01 |
| Triethylhexanoin | 10.00 | D-Asp | 0.01 |
| Isopropyl palmitate | 10.00 | D-Lys | 0.01 |
| Octyldodecyl myristate | 10.00 | D-Trp | 0.01 |
| PEG-20 glyceryl triisostearate | 10.00 | D-allo-Ile | 0.01 |
| | | D-Asn | 0.01 |
| D-Arg | 0.01 | D-Phe | 0.01 |
| D-His | 0.01 | D-Ala | 0.01 |
| D-Leu | 0.01 | D-Gln | 0.01 |
| D-Pro | 0.01 | D-Glu | 0.01 |
| D-Ser | 0.01 | Purified water | Remainder |
| D-Tyr | 0.01 | | |

### (Prescription Example 11; cleansing foam)

**[Table 16]**

| Name of component | Amount (parts by mass) | Name of component | Amount (parts by mass) |
|---|---|---|---|
| Sodium lauroyl glutamate | 35.00 | D-allo-Thr | 0.10 |
| Potassium myristate | 5.00 | D-Asp | 0.10 |
| Cocamide MEA | 1.00 | D-Lys | 0.10 |
| BG | 10.00 | D-Trp | 0.10 |
| Dipropylene glycol | 20.00 | D-allo-Ile | 0.10 |
| PVP | 0.30 | D-Asn | 0.10 |
| D-Arg | 0.10 | D-Phe | 0.10 |
| D-His | 0.10 | D-Ala | 0.10 |
| D-Leu | 0.10 | D-Gln | 0.10 |
| D-Pro | 0.10 | D-Glu | 0.10 |
| D-Ser | 0.10 | Talc | 1.00 |
| D-Tyr | 0.10 | Perfume | Proper amount |
| D-Val | 0.10 | Purified water | Remainder |

### (Prescription Example 12; body shampoo)

**[Table 17]**

| Name of component | Amount (parts by mass) | Name of component | Amount (parts by mass) |
|---|---|---|---|
| Sodium laureth sulfate | 3.00 | D-Pro | 0.01 |
| Cocamidopropyl betaine | 2.00 | D-Ser | 0.01 |
| Lauric acid | 6.52 | D-Tyr | 0.01 |
| Myristic acid | 5.25 | D-Val | 0.01 |
| Palmitic acid | 3.90 | D-allo-Thr | 0.01 |
| Stearic acid | 0.80 | D-Asp | 0.01 |
| Glycol distearate | 2.50 | D-Lys | 0.01 |
| PG | 3.50 | D-Trp | 0.01 |
| Cocamide MEA | 0.50 | D-allo-Ile | 0.01 |
| Cocamide methyl MEA | 1.50 | D-Asn | 0.10 |
| Sodium cocoamphoacetate | 0.20 | D-Phe | 0.01 |
| Hydroxypropyl methylcellulose | 0.20 | D-Ala | 0.10 |
| Polyquaternium-7 | 0.10 | D-Gln | 0.01 |
| PEG-7M | 0.05 | D-Glu | 0.01 |
| Guar hydroxypropyltrimonium chloride | 0.05 | Potassium hydroxide | 2.00 |
| | | Methylparaben | 0.20 |
| D-Arg | 0.01 | EDTA-2Na | 0.10 |
| D-His | 0.01 | Purified water | Remainder |
| D-Leu | 0.01 | | |

## Claims

1. A horny layer function improving agent comprising at least one D-amino acid selected from the group consisting of D-arginine, D-histidine, D-leucine, D-tyrosine, D-valine, D-allo-threonine, D-lysine, D-tryptophan, D-allo-isoleucine, D-asparagine, D-phenylalanine, D-glutamine, and D-threonine as an active ingredient.

2. The horny layer function improving agent according to claim 1, wherein the horny layer function improving is retention of a horny layer moisture content.

3. The horny layer function improving agent according to claim 1, wherein
the D-amino acid is at least one selected from the group consisting of D-arginine, D-histidine, D-leucine, D-valine, D-allo-threonine, D-lysine, D-tryptophan, D-allo-isoleucine, D-asparagine, D-phenylalanine, D-glutamine, and D-threonine, and
the horny layer function improving is inhibition of transepidermal water loss.

4. The horny layer function improving agent according to claim 1, wherein
the D-amino acid is at least one selected from the group consisting of D-histidine, D-leucine, D-tyrosine, D-lysine, D-tryptophan, D-allo-isoleucine, D-asparagine, D-phenylalanine, D-glutamine, and D-threonine, and
the horny layer function improving is a reduction in a wrinkle area rate.

5. A moisture retainability improving agent comprising at least one D-amino acid selected from the group consisting of D-arginine, D-histidine, D-leucine, D-tyrosine, D-valine, D-allo-threonine, D-lysine, D-tryptophan, D-allo-isoleucine, D-asparagine, D-phenylalanine, D-glutamine, and D-threonine as an active ingredient.

6. The moisture retainability improving agent according to claim 5, wherein the D-amino acid is at least one selected from the group consisting of D-histidine, D-leucine, D-lysine, D-tryptophan, D-allo-isoleucine, D-asparagine, and D-phenylalanine.

7. The moisture retainability improving agent according to claim 5 or 6, wherein the D-amino acid is at least one selected from the group consisting of D-histidine, D-leucine, D-lysine, D-tryptophan, D-allo-isoleucine, and D-phenylalanine.

8. A barrier function improving agent comprising at least one D-amino acid selected from the group consisting of D-arginine, D-histidine, D-leucine, D-valine, D-allo-threonine, D-lysine, D-tryptophan, D-allo-isoleucine, D-asparagine, D-phenylalanine, D-glutamine, and D-threonine as an active ingredient.

9. The barrier function improving agent according to claim 8, wherein the D-amino acid is at least one D-amino acid selected from the group consisting of D-histidine, D-leucine, D-lysine, D-tryptophan, D-allo-isoleucine, D-asparagine, and D-phenylalanine.

10. The barrier function improving agent according to claim 8 or 9, wherein the D-amino acid is at least one D-amino acid selected from the group consisting of D-histidine, D-leucine, D-lysine, D-tryptophan, D-allo-isoleucine, and D-phenylalanine.

11. A tiny wrinkle preventing or improving agent comprising at least one selected from the group consisting of D-histidine, D-leucine, D-tyrosine, D-lysine, D-tryptophan, D-allo-isoleucine, D-asparagine, D-phenylalanine, D-glutamine, and D-threonine as an active ingredient.

12. The tiny wrinkle preventing or improving agent according to claim 11, wherein the D-amino acid is at least one selected from the group consisting of D-leucine, D-lysine, D-tryptophan, and D-asparagine.

13. The tiny wrinkle preventing or improving agent according to claim 11 or 12, wherein the D-amino acid is at least one selected from the group consisting of D-leucine, D-lysine, and D-tryptophan.

14. An expression promoter of a gene of at least one protein selected from filaggrin, involucrin, loricrin, keratin 10, and transglutaminase 1 comprising at least one selected from the group consisting of D-lysine, D-tryptophan, D-allo-isoleucine, D-asparagine, and D-phenylalanine as an active ingredient.

15. A filaggrin production promoter comprising at least one selected from the group consisting of D-histidine, D-leucine, D-lysine, D-tryptophan, D-allo-isoleucine, D-phenylalanine, and D-threonine as an active ingredient.

16. A skin cosmetic or skin external preparation comprising the horny layer function improving agent according to any one of claims 1 to 4, the moisture retainability improving agent according to any one of claims 5 to 7, the barrier function improving agent according to any one of claims 8 to 10, the tiny wrinkle preventing or improving agent according to any one of claims 11 to 13, the expression promoter according to claim 14, or the production promoter according to claim 15.

17. The skin cosmetic or skin external preparation according to claim 16, further comprising at least one base selected from the group consisting of a polyol compound, a surfactant, a higher alcohol, a thickener, a chelating agent, an preservative, and a silicone.

18. The skin cosmetics or skin external preparation according to claim 16 or 17, wherein a dosage form is milky lotion, cream, skin lotion, or gel.

19. A method of evaluation of a skin by a D-amino acid, the method comprising:
measuring at least one selected from the group consisting of a horny layer moisture content, a wrinkle area rate, and a transepidermal water loss of a skin of a subject to determine at least one selected from the group consisting of moisture retainability, tiny wrinkle preventing or improving ability, and barrier function of the skin of the subject;
collecting a horny layer from a measurement site of the skin of the subject;
identifying at least one D-amino acid level contained in the horny layer; and
comparing a relation between the at least one selected from the group consisting of moisture retainability, barrier function, and tiny wrinkle preventing or improving ability of the skin and the D-amino acid level among results of the subject.

20. The method of evaluation according to claim 19, wherein the identification of the D-amino acid level is measurement of a D-amino acid content per content of protein contained in the horny layer.

21. The method of evaluation according to claim 19 or 20, wherein the quantification of the D-amino acid content is performed with a liquid chromatograph-tandem mass spectrometer.

22. The method of evaluation according to claim 20 or 21, wherein measurement of the content of protein is performed by the BCA method.

23. The method of evaluation according to any one of claims 19 to 22, wherein the D-amino acid comprises at least one selected from the group consisting of D-arginine, D-histidine, D-leucine, D-proline, D-serine, D-tyrosine, D-valine, D-allo-threonine, D-aspartic acid, D-lysine, D-tryptophan, D-allo-isoleucine, D-asparagine, D-phenylalanine, D-alanine, D-glutamine, D-glutamic acid, and D-methionine.

24. The method of evaluation according to claim 23, wherein
the subject is a woman in her twenties to thirties, and
the D-amino acid is at least one selected from the group consisting of D-arginine, D-histidine, D-leucine, D-proline, D-serine, D-tyrosine, D-valine, D-allo-threonine, D-aspartic acid, D-lysine, D-tryptophan, D-allo-isoleucine, D-asparagine, and D-phenylalanine,
the method evaluating at least moisture retainability.

25. The method of evaluation according to claim 23, wherein
the subject is a woman in her twenties to thirties, and
the D-amino acid is at least one selected from the group consisting of D-histidine, D-leucine, D-proline, D-serine, D-valine, D-allo-threonine, D-aspartic acid, D-tryptophan, D-allo-isoleucine, D-asparagine, and D-phenylalanine,
the method evaluating at least barrier function.

26. The method of evaluation according to claim 23, wherein
the subject is a woman in her forties to sixties, and
the D-amino acid is at least one selected from the group consisting of D-arginine, D-histidine, D-leucine, D-proline, D-valine, D-allo-threonine, D-aspartic acid, D-lysine, D-tryptophan, D-allo-isoleucine, D-asparagine, and D-glutamine,
the method evaluating at least moisture retainability.

27. The method of evaluation according to claim 23, wherein
the subject is a woman in her forties to sixties, and
the D-amino acid is at least one selected from the group consisting of D-arginine, D-histidine, D-leucine, D-proline, D-serine, D-valine, D-allo-threonine, D-lysine, D-tryptophan, D-allo-isoleucine, D-phenylalanine, and D-glutamine,
the method evaluating at least barrier function.

28. The method of evaluation according to claim 23, wherein
the subject is a woman in her twenties to thirties, and
the D-amino acid is at least one selected from the group consisting of D-leucine, D-tyrosine, D-aspartic acid, D-lysine, D-tryptophan, D-asparagine, and D-glutamine,
the method evaluating at least tiny wrinkle preventing or improving ability.

29. The method of evaluation according to claim 23, wherein
the subject is a woman in her forties to sixties, and
the D-amino acid is at least either D-aspartic acid or D-lysine,
the method evaluating at least tiny wrinkle preventing or improving ability.
